# EUROPEAN PATENT APPLICATION

(11) **EP 4 321 506 A1**
(43) Date of publication of application: **14.02.2024**
(21) Application number: 22189461.1
(22) Date of filing: 09.08.2022
(51) Int. Cl.: C07D 231/20, C07D 401/04, C07D 401/14, C07D 403/04, C07D 403/14, C07F 1/08, C07F 1/10, C07F 5/00, C07F 5/06, C07F 13/00, C07F 17/02

(54) **METAL COMPLEX, SEMICONDUCTOR LAYER COMPRISING A METAL COMPLEX AND ORGANIC ELECTRONIC DEVICE**

(71) Applicant: Novaled GmbH, 01099 Dresden (DE)
(72) Inventor: PINTER, Piermaria, 01099 Dresden (DE); UVAROV, Vladimir, 01099 Dresden (DE); HEGGEMANN, Ulrich, 01099 Dresden (DE); WILLMANN, Steffen, 01099 Dresden (DE)
(74) Representative: Michalski Hüttermann & Partner Patentanwälte mbB

(57) **Abstract**

The present invention relates to a metal complex, a semiconductor layer comprising the metal complex and an organic electronic device comprising at least one metal complex thereof.

## Description

### Technical Field

The present invention relates to a metal complex, a semiconductor layer comprising the metal complex and an organic electronic device comprising at least one metal complex thereof.

### Background Art

Electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode layer, a hole injection layer HIL, a hole transport layer HTL, an emission layer EML, an electron transport layer ETL, and a cathode layer, which are sequentially stacked on a substrate. In this regard, the HIL, the HTL, the EML, and the ETL are thin films formed from organic compounds.

When a voltage is applied to the anode and the cathode, holes injected from the anode move to the EML, via the HIL and HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has low operating voltage, excellent efficiency and/or a long lifetime.

Performance of an organic light emitting diode may be affected by characteristics of the hole injection layer, and among them, may be affected by characteristics of the hole transport compound and the metal complexes which are contained in the hole injection layer.

US 2015200374 A relates to a hole injection layer consisting of quadratic planar mononuclear transition metal complexes such as copper 2+ complexes, for example, which are embedded into a hole-conducting matrix.

WO16188604 A1 relates to a composition at least one hole-transport or/and one hole-injection material and at least one metal complex as a p-dopant.

Performance of an organic light emitting diode may be affected by characteristics of the semiconductor layer, and among them, may be affected by characteristics of metal complexes which are also contained in the semiconductor layer.

There remains a need to improve performance of a metal complex, suitable for use as semiconductor materials, semiconductor layers, as well as electronic devices thereof, in particular to achieve improved operating voltage stability over time through improving the characteristics of the metal complex comprised therein.

Further there remains a need to improve performance of a metal complex, suitable for use as semiconductor materials, with improved thermal properties and/or solubility in organic solvents.

Further there remains a need to improve performance of electronic devices by providing semiconductor layers, such as hole injection layers, with improved performance, in particular to achieve improved operating voltage through improving the characteristics of the semiconductor layer, such as hole injection layer, and the electronic device.

### DISCLOSURE

An aspect of the present invention provides a metal complex of formula (I)

M^{n_{⊕}}(L^{⊝})ₙ(AL)ₘ (I),

wherein
M is a metal ion;
n is the valency of M and selected from 1 to 4;
L has formula (II) 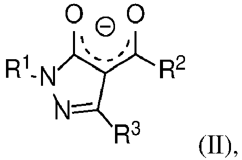
   - R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
   - R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
   wherein
   at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
   at least one of R¹, R² and R³ or the group of R¹, R² and R³ comprises at least three atoms selected from the group consisting of halogen, Cl, F or N;
wherein
   - AL: is an ancillary ligand which coordinates to the metal M;
   - m: is an integer selected from 0 to 2.

### Definitions

It should be noted that throughout the application and the claims that the metal complex or metal complex of formula (I) is also referred to as compound.

It should be noted that throughout the application and the claims any R¹, R², R³, L and M. always refer independently from each other to the same moieties according to their definition, unless otherwise noted.

In the present specification, when a definition is not otherwise provided, "substituted" refers to a substituted selected from substituted or unsubstituted C₁ to C₁₂ alkyl, partially or fully fluorinated C₁ to C₁₂ alkyl, substituted or unsubstituted C₆ to C₁₉ aryl, and substituted or unsubstituted C₂ to C₂₀ heteroaryl, wherein the substituents are selected from the substituents of the substituted or unsubstituted C₁ to C₁₂ alkyl, substituted or unsubstituted C₆ to C₁₉ aryl, and substituted or unsubstituted C₂ to C₂₀ heteroaryl are selected are independently selected from halogen, CI, F, CN, partially or perfluorinated C₁ to C₈ alkyl, partially or perfluorinated C₁ to C₈ alkoxy.

In the present specification, "aryl group" and "aromatic rings" refers to a hydrocarbyl group which may be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under "heteroaryl" and "heteroaromatic", it is especially where suitable understood a group derived by formal abstraction of one ring hydrogen from a heterocyclic aromatic ring in a metal complex comprising at least one such ring.

The term "non-heterocycle" is understood to mean a ring or ring-system comprising no hetero-atom as a ring member.

The term "heterocycle" is understood to mean that the heterocycle comprises at least one ring comprising one or more hetero-atoms. A heterocycle comprising more than one ring means that all rings comprising a hetero-atom or at least one ring comprising a hetero atom and at least one ring comprising C-atoms only and no hetero atom. A C₂ heteroaryl group means that an heteroaryl ring comprises two C-Atoms and the other atoms are hetero-atoms.

Under heterocycloalkyl, it is especially where suitable understood a group derived by formal abstraction of one ring hydrogen from a saturated cycloalkyl ring in a compound comprising at least one such ring.

The term "aryl" having at least 9 C-atoms may comprise at least one fused aryl ring. The term "heteroaryl" having at least 9 atoms may comprise at least one fused heteroaryl ring fused with a heteroaryl ring or fused with an aryl ring.

The term "fused aryl rings" or "condensed aryl rings" is understood the way that two aryl rings are considered fused or condensed when they share at least two common sp²-hybridized carbon atoms.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the present specification, when a definition is not otherwise provided, "substituted" refers to one substituted with a H, deuterium, halogen, Cl, F, CN, partially or perfluorinated C₁ to C₈ alkyl, partially or perfluorinated C₁ to C₈ alkoxy.

In the present specification "substituted aryl" refers for example to a C₆ to C₁₉ aryl or C₆ to C₁₈ aryl that is substituted with one or more substituents, wherein the substituent may be substituted with none, one or more substituents, preferably the substituent may be selected from H, deuterium, halogen, Cl, F, CN, partially or perfluorinated C₁ to C₈ alkyl, partially or perfluorinated C₁ to C₈ alkoxy.

Correspondingly, in the present specification "substituted heteroaryl" refers to a substitution with one or more substituents, which themselves may be substituted with one or more substituents, preferably the substituent may be selected from H, deuterium, halogen, Cl, F, CN, partially or perfluorinated C₁ to C₈ alkyl, partially or perfluorinated C₁ to C₈ alkoxy..

In the present specification, when a definition is not otherwise provided, a substituted heteroaryl group with at least 2 C-ring atoms may be substituted with one or more substituents. For example, a substituted C₂ heteroaryl group may have 1 or 2 substituents.

A substituted aryl group with at least 6 ring atoms may be substituted with 1, 2, 3, 4 or 5 substituents.

A substituted heteroaryl group may comprise at least 5 or 6 ring atoms. A substituted heteroaryl group that may comprise at least 5 or 6 ring atoms may be substituted with 1, 2, 3 or 4 substituents, if the heteroaryl group comprises one hetero atom and five C-atoms, or it may be substituted with 1, 2 or 3 substituents, if the heteroaryl group with at least 6 ring atoms comprises two hetero atom and four C-atoms, or may be substituted with 1 or 2 substituents, if the heteroaryl group with at least 6 ring atoms comprises three hetero atoms and three C-atoms, wherein the substituent is bonded to the C-ring atoms only.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, tert-butyl, cyclohexyl.

Specific examples of the alkyl group may be a methyl group, an ethyl group, a propyl group, an iso-propyl group, a butyl group, an iso-butyl group, a sec-butyl group, a tert-butyl group, a pentyl group, a branched pentyl group, a hexyl group, a cyclopropyl group, a cyclobutyl group, a cyclopentyl group, a cyclohexyl group, an adamantly group and the like.

In the present specification, when a definition is not otherwise provided, a "substituted alkyl group" may refer to a linear, branched or cyclic substituted saturated aliphatic hydrocarbyl group. The substituted alkyl group may be a linear, branched or cyclic C₁ to C₁₂ alkyl group. More specifically, the substituted alkyl group may be a linear, branched or cyclic substituted C₁ to C₁₀ alkyl group or a linear, branched or cyclic substituted C₁ to C₆ alkyl group. For example, a linear, branched or cyclic substituted C₁ to C₄ alkyl group includes 1 to 4 carbons in the alkyl chain, and may be selected from methyl, ethyl, propyl, iso-propyl, n-butyl, iso-butyl, sec-butyl, tert-butyl and cyclohexyl. The substituents may be selected from halogen, F, Cl, CN, OCH₃, OCF₃.

The term "hetero" is understood the way that at least one carbon atom, in a structure which may be formed by covalently bound carbon atoms, is replaced by another polyvalent atom. Preferably, the heteroatoms are selected from B, Si, N, P, O, S; further preferred from N, P, O, S and most preferred N.

In the present specification, when a substituent is not named, the substituent may be a H.

The term "charge-neutral" means that the group L is overall electrically neutral.

In the context of the present invention, "different" means that the metal complexes do not have an identical chemical structure.

The term "free of", "does not contain", "does not comprise" does not exclude impurities which may be present in the metal complex prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The term "at least two anode sub-layers" is understood to mean two or more anode sub-layers, for example two or three anode sub-layers.

The terms cathode, cathode layer and cathode electrode are used synonymously.

The term "hole injection layer" is understood to mean a layer which improves charge injection from the anode layer into further layers in the electronic device or from further layers of the electronic device into the anode.

The term "hole transport layer" is understood to mean a layer which transports holes between the hole injection layer and further layers arranged between the hole injection layer and the cathode layer.

The operating voltage U is measured in Volt.

In the context of the present specification the term "essentially non-emissive" or "non-emissive" means that the contribution of the metal complex of formula (I) or the hole injection layer comprising a metal complex of formula (I), to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

In the context of the present invention, the term "sublimation" may refer to a transfer from solid state to gas phase or from liquid state to gas phase.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO level of the matrix compound of the hole injection layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "- "symbol. According to one embodiment of the present invention, the HOMO level of the matrix compound of the hole injection layer may be calculated by quantum mechanical methods.

### Advantageous Effects

Surprisingly, it was found that the electronic device according to the invention solves the problem underlying the present invention by enabling electronic devices, such as organic light-emitting diodes, in various aspects superior over the electronic devices known in the art, in particular with respect to operating voltage.

Additionally, it was found that the problem underlying the present invention may be solved by providing metal complexes which may be suitable for deposition through vacuum thermal evaporation under conditions suitable for mass production. In particular, the rate onset temperature of the metal complexes of formula (I) of the present invention may be in a range suitable for mass production. In addition the metal complexes of formula (I) of the present invention shows improved thermal properties and/or solubility in organic solvents.

The metal complex of formula (I) is non-emissive. In the context of the present specification the term "essentially non-emissive" or "non-emissive" means that the contribution of the metal complex of formula (I) to the visible emission spectrum from an electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

### M of the metal complex of formula (I)

The term "M" represents a metal ion. According to one embodiment, M is a metal ion selected from an alkali, alkaline earth, transition, rare earth metal or group III to V metal, preferably M is a metal ion selected from transition or group III to V metal; preferably M is a metal ion selected from Li(I), K(I), Rb(I), Cs(I), Ag(I), Cu(II), Zn(II), Pd(II), Ir(III), Al(III), Sc(III), Mn(III), Ru(III), In(III), Y(III), Eu(III), Fe(III), V(IV), Zr(IV), Hf(IV) and Ce(IV); more preferred M is a metal ion selected from Li(I), K(I), Ag(I), Cu(II), Zn(II), Al(III), Sc(III), Mn(III), Fe(III), Zr(IV), and Ce(IV); also preferred M is Cu(II) or Fe(III), and further more preferred M is Cu(II); wherein the number in brackets denotes the oxidation state.

According to one embodiment M is a metal ion may be selected from Cu, Fe, Mn, Al, Hf or Zr, preferably Cu(II), Fe(III), Mn(III), Al(III), Hf(IV) or Zr(IV).

According to one embodiment M is a metal ion may be selected from Ce, preferably Ce(IV).

### Ligand L of formula (I)

The term "L" is represented by formula (II) 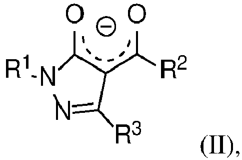 wherein
- R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
- R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprises at least three atoms selected from the group consisting of halogen, Cl, F or N.

According to one embodiment, wherein the ligand L in metal complex of formula (I) may be selected from a group comprising at least 11 carbon atoms and at most 20 carbon atoms, alternatively at least 12 carbon atoms and at most 19 carbon atom.

According to one embodiment, wherein the ligand L in metal complex of formula (I) may be selected from a group comprising at least 11 carbon atoms and at most 20 carbon atoms, alternatively at least 12 carbon atoms and at most 19 carbon atom; and two oxygen atoms and at least one to four nitrogen atoms, or two oxygen atoms and two nitrogen atoms, or two oxygen atoms and three nitrogen atoms, or two oxygen atoms and four nitrogen atoms.

According to one embodiment, wherein the ligand L in metal complex of formula (I) may be selected from a group comprising at least 11 carbon atoms and at most 20 carbon atoms, alternatively at least 12 carbon atoms and at most 19 carbon atom; and two oxygen atoms and at least one to four nitrogen atoms, or two oxygen atoms and two nitrogen atoms, or two oxygen atoms and three nitrogen atoms, or two oxygen atoms and four nitrogen atoms; and at least one or more groups selected from halogen, F, CN, perfluorinated C₁ to C₁₆ alkyl.

According to one embodiment, wherein the ligand L in metal complex of formula (I) may be selected from a group comprising at least 11 carbon atoms and at most 20 carbon atoms, alternatively at least 12 carbon atoms and at most 19 carbon atom; and two oxygen atoms and at least one to four nitrogen atoms, or two oxygen atoms and two nitrogen atoms, or two oxygen atoms and three nitrogen atoms, or two oxygen atoms and four nitrogen atoms; and at least one to five groups selected from halogen, F, CN, perfluorinated C₁ to C₃ alkyl.

### n valency of M

The term "n" is the valency of M and selected from 1 to 4, preferably n = 1, 2, 3 or 4, further preferred n = 3 or 4. In addition preferred n = 1 or 2.

According to one embodiment "n" is an integer selected from 2, 3 and 4, which corresponds to the valency of M. According to one embodiment "n" is an integer selected from 2 or 3, which corresponds to the valency of M. According to one embodiment "n" is selected 2. According to another embodiment "n" is selected 4. According to another embodiment "n" is selected 3. According to another embodiment "n" is selected 4.

### The term "m"

The term "m" is an integer selected from 0 to 2, which corresponds to the oxidation number of M. According to one embodiment "m" is an integer selected from 0 or 1. According to another embodiment "m" is an integer selected from 1. According to another embodiment "m" is an integer selected from 2. Preferably "m" is an integer and may be selected from 0.

According to another embodiment wherein n = 2 or 3; and/or m is an integer selected from 0 or 1, preferably 0.

### Embodiments

The metal complex of formula (I) can be also named metal complex or metal acetylacetonate complex.

According to one embodiment, the metal complex of formula (I) may have a molecular weight Mw of ≥ 287 g/mol and ≤ 2000 g/mol, preferably a molecular weight Mw of ≥ 400 g/mol and ≤ 1500 g/mol, further preferred a molecular weight Mw of ≥ 580 g/mol and ≤ 1500 g/mol, in addition preferred a molecular weight Mw of ≥ 580 g/mol and ≤ 1400 g/mol, in addition preferred a molecular weight Mw of ≥ 580 g/mol and ≤ 1100 g/mol.

According to one embodiment, wherein the metal complex of formula (I)

M^{n_{⊕}}(L^{⊝})ₙ(AL)ₘ (I),

wherein
M is a metal ion;
n is the valency of M and selected from 1 to 4;
L has formula (II) 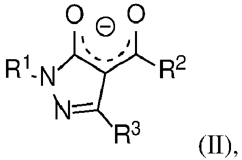
   - R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
   - R³: is a C₁ to C₃ alkyl, substituted or unsubstituted C₆ to C₁₂ aryl, substituted or unsubstituted C₃ to C₉ heteroaryl or CN, preferably CH₃ or CN;
   wherein
   at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
   at least one of R¹, R² and R³ or the group of R¹, R² and R³ comprises at least three atoms selected from the group consisting of halogen, Cl, F or N;
wherein
AL is an ancillary ligand which coordinates to the metal M;
m is an integer selected from 0 to 2.

According to one embodiment, wherein the metal complex of formula (I)

M^{n_{⊕}}(L^{⊝})ₙ(AL)ₘ (I),

wherein
M is a metal ion;
n is the valency of M and selected from 1 to 4;
L has formula (II) 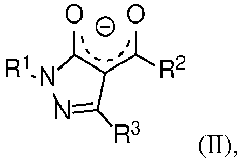
   - R¹ and R²: are independently selected from substituted C₆ to C₁₂ aryl or substituted C₃ to C₉ heteroaryl;
   - R³: is a C₁ to C₃ alkyl, substituted or unsubstituted C₆ to C₁₂ aryl, substituted or unsubstituted C₃ to C₉ heteroaryl or CN, preferably CH₃ or CN;
   wherein
   at least one of the substituents of the substituted C₆ to C₁₂ aryl or substituted C₃ to C₉ heteroaryl are independently selected from F, CN, partially or perfluorinated C₁ to C₃ alkyl, partially or perfluorinated C₁ to C₃ alkoxy;
wherein
   at least one of R¹, R² and R³ or the group of R¹, R² and R³ comprises at least three atoms selected from the group consisting of halogen, Cl, F or N;
wherein
AL is an ancillary ligand which coordinates to the metal M;
m is an integer selected from 0 to 2.

According to one embodiment, wherein the metal complex of formula (I)

M^{n_{⊕}}(L^{⊝})ₙ(AL)ₘ (I),

wherein
M is a metal ion;
n is the valency of M and selected from 1 to 4;
L has formula (II) 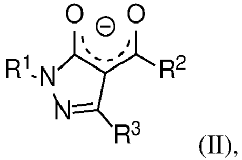
   - R¹ and R²: are substituted C₆ aryl or substituted 6-member C₄ to C₅ heteroaryl;
   - R³: is a C₁ to C₃ alkyl, substituted or unsubstituted C₆ aryl, substituted or unsubstituted 6-member C₄ to C₅ heteroaryl or CN, preferably CH₃ or CN;
   wherein
   at least one of the substituents of the substituted C₆ aryl or substituted 6-member C₄ to C₅ heteroaryl are independently selected from F, CN, partially or perfluorinated C₁ to C₃ alkyl, partially or perfluorinated C₁ to C₃ alkoxy;
wherein
   at least one of R¹, R² and R³ or the group of R¹, R² and R³ comprises at least three atoms selected from the group consisting of halogen, Cl, F or N;
wherein
AL is an ancillary ligand which coordinates to the metal M;
m is an integer selected from 0 to 2.

According to one embodiment, wherein the metal complex of formula (I)

M^{n_{⊕}}(L^{⊝})ₙ(AL)ₘ (I),

wherein
M is a metal ion;
n is the valency of M and selected from 1 to 4;
L has formula (II) 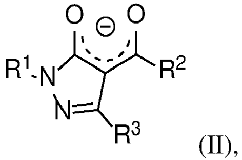
   - R¹ and R²: are substituted C₆ aryl or substituted 6-member C₄ to C₅ heteroaryl;
   - R³: is a C₁ to C₃ alkyl, substituted or unsubstituted C₆ aryl, substituted or unsubstituted 6-member C₄ to C₅ heteroaryl or CN, preferably CH₃ or CN;
   wherein
   at least one of the substituents of the substituted C₆ aryl or substituted 6-member C₄ to C₅ heteroaryl are independently selected from F, CN, partially or perfluorinated C₁ to C₃ alkyl, partially or perfluorinated C₁ to C₃ alkoxy;
wherein
   at least one of R¹, R² and R³ or the group of R¹, R² and R³ comprises at least three atoms selected from the group consisting of halogen, Cl, F or N;
wherein
AL is an ancillary ligand which coordinates to the metal M;
m is an integer selected from 0 to 2; and
wherein the the metal complex of formula (I) comprises at least one or two 6-member rings and at least one 5-member ring.

According to one embodiment, wherein the metal complex of formula (I):

M^{n_{⊕}}(L^{⊝})ₙ(AL)ₘ (I),

wherein
- M: is a metal ion selected from a main group metal or transition metal, preferably Cu, Fe, Mn, Al, Hf, Zr;
- n: is the valency of M and selected from 2 or 3;
- L: has formula (II) 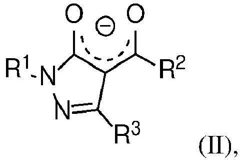
- R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
- R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
   at least one of R¹, R² and R³ or the group of R¹, R² and R³ comprises at least three atoms selected from the group consisting of halogen, Cl, F or N;
wherein
   the group consisting of R¹, R² and R³ comprises in addition:
      - at least two moieties selected from CF₃ or CN; or
      - at least one moiety selected from CF₃ or CN and one substituted or unsubstituted 6-membered heteroaryl; or
      - R³ is CN;
         - AL: is an ancillary ligand which coordinates to the metal M;
         - m: is an integer selected from 0 to 2.

According to one embodiment, wherein the metal complex of formula (I):

M^{n_{⊕}}(L^{⊝})ₙ(AL)ₘ (I),

wherein
- M: is a Ce ion;
- n: is the valency of M and is 4;
- L: has formula (II) 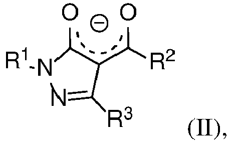
- R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
- R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprise at least three atoms
selected from the group consisting of halogen, Cl, F or N;
AL is an ancillary ligand which coordinates to the metal M;
m is an integer selected from 0 to 2.

According to one embodiment, wherein the metal complex of formula (I):

M^{n_{⊕}}(L^{⊝})ₙ(AL)ₘ (I),

wherein
- M: is a metal ion;
- n: is the valency of M and selected from 1 to 4;
- L: has formula (II) 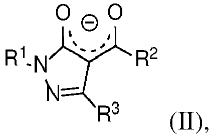
- R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl that comprises at least one substituted or unsubstituted 6-membered heteroaryl;
- R³: is selected from partially or perfluorinated C₁ to C₄ alkyl or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, CI, F, CN, partially or perfluorinated C₁ to C₈ alkyl, partially or perfluorinated C₁ to C₈ alkoxy;
wherein
- AL: is an ancillary ligand which coordinates to the metal M;
- m: is an integer selected from 0 to 2. According to one embodiment, wherein the metal complex of formula (I):

M^{n_{⊕}}(L^{⊝})ₙ(AL)ₘ (I),

wherein
- M: is a metal ion;
- n: is the valency of M and selected from 1 to 4;
- L: has formula (II) 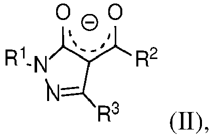
- R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl;
- R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, CI, F, CN, partially or perfluorinated C₁ to C₈ alkyl, partially or perfluorinated C₁ to C₈ alkoxy;
wherein
at least one of R¹, R² and R³ or the group of R¹, R² and R³ comprises a substituted or unsubstituted C₂ to C₂₀ heteroaryl that comprises at least one 6 membered heteroaryl ring; wherein
- AL: is an ancillary ligand which coordinates to the metal M;
- m: is an integer selected from 0 to 2.

According to one embodiment of the metal complex of formula (I), wherein at least one R¹ or R³, or R¹ and R³ are selected from a substituted C₂ to C₂₀ heteroaryl group comprising at least 6 ring-forming atoms, wherein the C₂ to C₂₀ heteroaryl group comprising at least 6 ring-forming atoms is selected from pyridyl, pyrimidinyl, pyrazinyl, triazinyl.

According to one embodiment of the metal complex of formula (I), wherein R¹ is selected from unsubstituted or substituted phenyl, substituted pyridyl, substituted pyrimidinyl, substituted pyrazinyl, substituted triazinyl, wherein the substituents of the substituted phenyl, substituted pyridyl, substituted pyrimidinyl, substituted pyrazinyl, or substituted triazinyl are selected from CN, CF₃, C₂F₅, C₃F₇ or C₄F₉.

According to one embodiment of the metal complex of formula (I), wherein R¹ is selected from unsubstituted or substituted phenyl, substituted pyridyl, substituted pyrimidinyl, substituted pyrazinyl, substituted triazinyl, wherein at least two or three of the substituents of the substituted phenyl, substituted pyridyl, substituted pyrimidinyl, substituted pyrazinyl, or substituted triazinyl are selected from CN, CF₃, C₂F₅, C₃F₇ or C₄F₉.

According to one embodiment of the metal complex of formula (I), wherein R² is selected from substituted pyridyl, substituted pyrimidinyl, substituted pyrazinyl, substituted triazinyl, CF₃, C₂F₅, C₃F₇ or C₄F₉, wherein the substituents of the substituted pyridyl, substituted pyrimidinyl, substituted pyrazinyl, or substituted triazinyl are selected from CF₃, C₂F₅, C₃F₇ or C₄F₉.

According to one embodiment of the metal complex of formula (I), wherein R² is selected from substituted pyridyl, substituted pyrimidinyl, substituted pyrazinyl, substituted triazinyl, CF₃, C₂F₅, C₃F₇ or C₄F₉, wherein at least two or three of the substituents of the substituted pyridyl, substituted pyrimidinyl, substituted pyrazinyl, or substituted triazinyl are selected from CF₃, C₂F₅, C₃F₇ or C₄F₉.

According to one embodiment of the metal complex of formula (I), wherein R² is selected from CF₃, C₂F₅, C₃F₇ or C₄F₉.

According to one embodiment of the metal complex of formula (I), wherein R³ is selected from unsubstituted or substituted phenyl, CH₃, or CN, wherein the substituents of the substituted phenyl are selected from CN, CF₃, C₂F₅, C₃F₇ or C₄F₉, preferably CF₃.

According to one embodiment of the metal complex of formula (I), wherein at least one of R¹, R² and R³ or the group of R¹, R² and R³ comprises at least three atoms selected from the group consisting of halogen, Cl, F or N.

According to one embodiment of the metal complex of formula (I), wherein R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl that comprises at least one substituted or unsubstituted 6-membered heteroaryl; and R³ is selected from partially or perfluorinated C₁ to C₄ alkyl or CN, preferably CF₃.

According to one embodiment of the metal complex of formula (I), wherein the group consisting of R¹, R² and R³ comprises at least two moieties selected from CF₃ or CN; or wherein the group consisting of R¹, R² and R³ comprises at least one moiety selected from CF₃ or CN and one substituted or unsubstituted 6-membered heteroaryl; or R³ is CN.

According to one embodiment of the metal complex of formula (I), wherein at least one of the group consisting of R¹, R² and R³ is selected from CN, CH₃, CF₃, C₂F₅, C₃F₇ or C₄F₉, preferably CN, CH₃, CF₃, C₂F₅ or C₃F₇; more preferred CN, CH₃ or CF₃.

According to one embodiment of the metal complex of formula (I), wherein the metal complex of formula (I) comprises at least one CF₃ group, preferably at least two CF₃ groups, further preferred at least three CF₃ groups, in addition preferred at least four CF₃ groups, or at least two CF₃ groups and at least one C₂F₅, or at least two CF₃ groups and at least one group selected from CN, F or CH₃.

According to one embodiment of the metal complex of formula (I), wherein at least one R¹ and/or R² is selected from a substituted C₆ to C₁₉ aryl or substituted C₃ to C₂₀ heteroaryl group, wherein at least one substituent of the substituted C₆ to C₁₉ aryl or substituted C₃ to C₂₀ heteroaryl group is selected from CN or partially or fully fluorinated C₁ to C₁₂ alkyl, preferably partially or fully fluorinated C₁ to C₄ alkyl.

According to one embodiment of the metal complex of formula (I), wherein at least one R¹ and/or R² is selected from a substituted C₆ to C₁₉ aryl or substituted C₃ to C₂₀ heteroaryl group, wherein at least two substituent of the substituted C₆ to C₁₉ aryl or substituted C₃ to C₂₀ heteroaryl group is selected from CN or partially or fully fluorinated C₁ to C₁₂ alkyl, preferably partially or fully fluorinated C₁ to C₄ alkyl.

According to one embodiment of the metal complex of formula (I), wherein the metal complex of formula (I) comprises at least one CF₃ group, preferably at least two CF₃ groups, further preferred at least three CF₃ groups, in addition preferred at least four CF₃ groups, or at least two CF₃ groups and at least one C₂F₅, or at least two CF₃ groups and at least one group selected from CN, F or CH₃.

According to one embodiment of the metal complex of formula (I), wherein at least one R¹ and/or R² are selected from a substituted C₃ to C₂₀ heteroaryl group comprising at least 6 ring-forming atoms, wherein
- the substituted heteroaryl group comprises at least 2 to 6 N atoms, preferably 3 to 5 N atoms, and more preferred 2 N atom; and/or
- the heteroaryl group of the substituted heteroaryl group is a six-membered ring, comprise 1, 2 or 3 hetero atoms, wherein preferably the hetero atom is N.

According to one embodiment of the metal complex of formula (I), wherein at least one R¹ and/or R² are selected from a substituted C₃ to C₂₀ heteroaryl group comprising at least 6 ring-forming atoms, wherein
- the substituted heteroaryl group comprises at least 2 to 6 N atoms, preferably 3 to 5 N atoms, and more preferred 2 N atom; and/or
- the heteroaryl group of the substituted heteroaryl group is a six-membered ring, comprise 1, 2 or 3 hetero atoms, wherein preferably the hetero atom is N.

According to one embodiment, wherein at least one R¹ and/or R² is selected from a substituted C₃ to C₂₀ heteroaryl group, wherein the heteroaryl group is selected from pyridyl, pyrimidinyl pyrazinyl, or triazinyl.

According to one embodiment, wherein at least one R¹ and/or R² are selected from a substituted C₃ to C₂₀ heteroaryl group, wherein the C₃ to C₂₀ heteroaryl group is selected from pyridyl, pyrimidinyl, pyrazinyl, triazinyl.

According to one embodiment, wherein at least one R¹ and/or R² is selected from a substituted C₃ to C₂₀ heteroaryl group, wherein the at least one substituent of the substituted heteroaryl group is selected from the group comprising halogen, F, Cl, CN, partially or fully fluorinated C₁ to C₆ alkyl, preferably from the group comprising halogen, F, Cl, CN, partially or fully fluorinated C₁ to C₆ alkyl, further preferred from the group comprising halogen, F, Cl, CN, partially or fully fluorinated C₁ to C₄ alkyl; and more preferred from the group comprising F, CN, partially or fully fluorinated C₁ to C₆ alkyl; also preferred F, CN, partially or fully fluorinated C₁ to C₆ alkyl, also preferred halogen, F, Cl, CN, partially or fully fluorinated C₁ to C₄ alkyl; and more preferred from the group comprising at least one CN, at least one CF₃ group, and/or at least two F atoms.

According to one embodiment, wherein at least one R¹ and/or R² are selected from a substituted C₃ to C₂₀ heteroaryl group, wherein at least one substituent is selected from halogen, F, Cl, CN, partially or fully fluorinated C₁ to C₆ alkyl, and one R¹ and/or R² is selected from substituted or unsubstituted C₁ to C₁₂ alkyl, wherein the at least one substituent is selected from halogen, F, Cl, CN, partially or fully fluorinated C₁ to C₆ alkyl.

According to one embodiment, wherein one R¹ is selected from a substituted C₃ to C₂₀ heteroaryl group, wherein at least one, two or three substituents are selected from CF₃ group.

According to one embodiment, wherein R² is selected from a substituted C₃ to C₂₀ heteroaryl group, wherein at least one, two or three substituents are selected from CF₃ group and/or CN group.

According to one embodiment, wherein R¹ is selected from a substituted C₆ to C₁₉ aryl group, wherein at least one, two or three substituents are selected from CF₃ group.

According to one embodiment, wherein R² is selected from a substituted C₆ to C₁₉ aryl group, wherein at least one, two or three substituents are selected from CF₃ group and/or CN group.

According to one embodiment, wherein R¹ is selected from a substituted pyridine group, wherein at least one, two or three substituents are selected from CF₃ group.

According to one embodiment, wherein R² is selected from a substituted pyridine group, wherein at least one, two or three substituents are selected from CF₃ group and/or CN group.

According to one embodiment, wherein R¹ is selected from a substituted phenyl group, wherein at least one, two or three substituents are selected from CF₃ group.

According to one embodiment, wherein R² is selected from a substituted phenyl group, wherein at least one, two or three substituents are selected from CF₃ group and/or CN group.

According to one embodiment, wherein R¹ is selected from a substituted phenyl group, wherein at least one, two or three substituents are selected from CF₃ group; and wherein R³ is selected from a substituted phenyl group, wherein at least one, two or three substituents are selected from CF₃ group and/or CN group.

According to one embodiment, wherein R¹ and/or R² maybe not selected from a substituted or unsubstituted C₆ to C₁₉ aryl group or a substituted or unsubstituted C₆ to C₁₈ aryl group.

According to one embodiment, wherein R¹ and R² may be not selected from a unsubstituted aryl and/or unsubstituted heteroaryl group.

According to one embodiment, wherein Formula (I) may not comprise an unsubstituted aryl and/or unsubstituted heteroaryl group.

According to one embodiment, wherein Formula (I) may not comprises a substituted aryl group.

According to one embodiment, wherein Formula (I) may not comprises a substituted heteroaryl group.

According to one embodiment, wherein at least one of the group consisting of R¹, R² and R³ of Formula (I) is selected from a substituted or unsubstituted C₆ to C₁₉ aryl or a substituted C₂ to C₂₀ heteroaryl group comprising at least 6 ring-forming atoms, and is selected from the following Formulae D1 to D71: 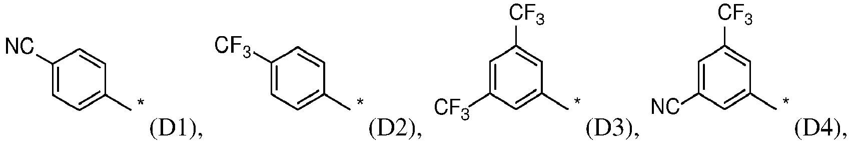 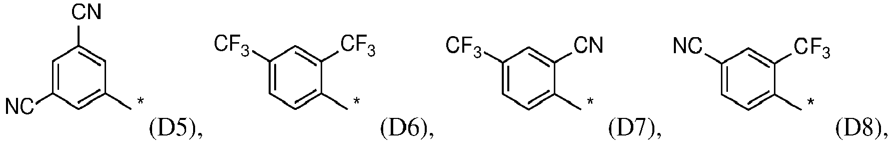 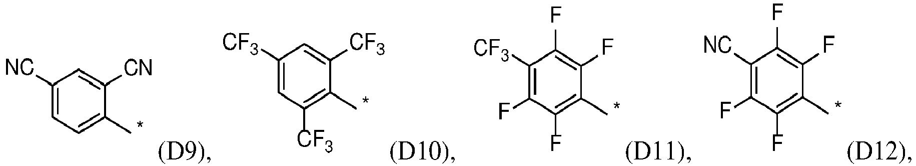 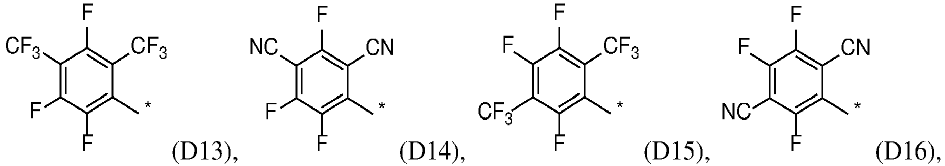 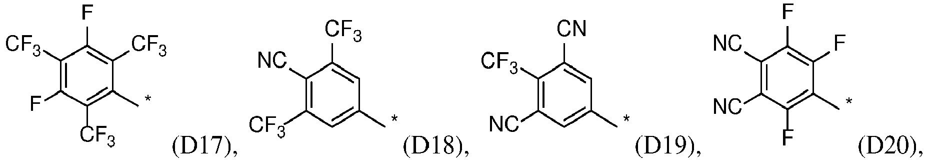 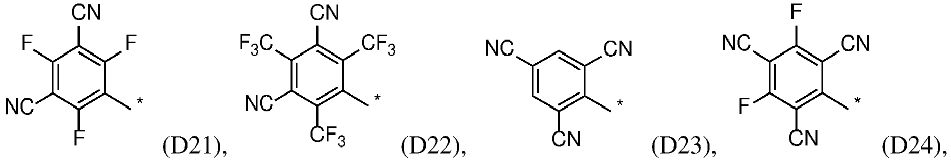 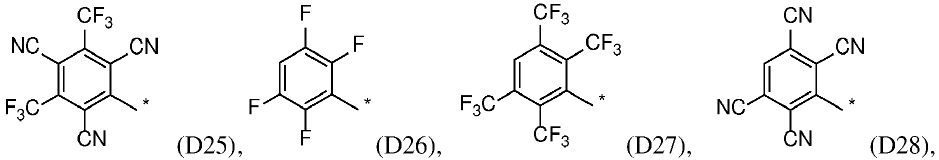 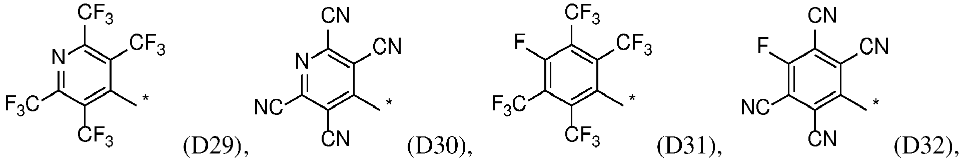 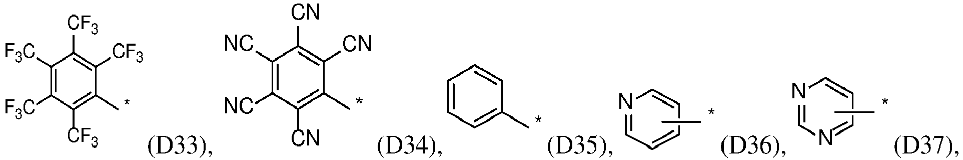 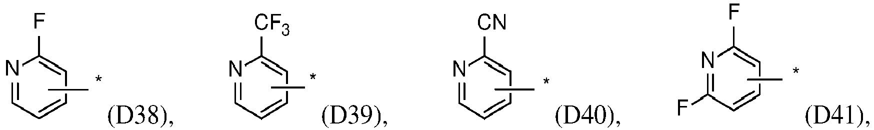 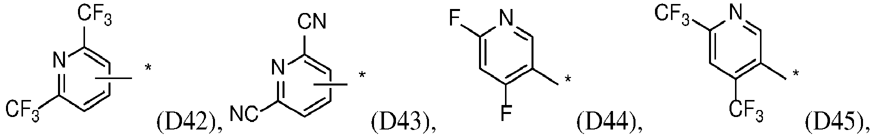 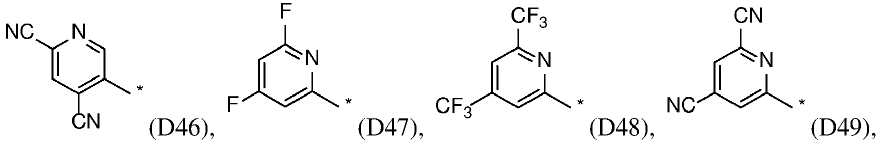 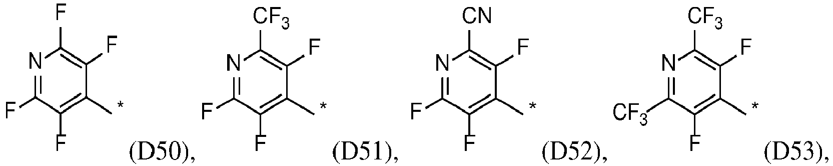 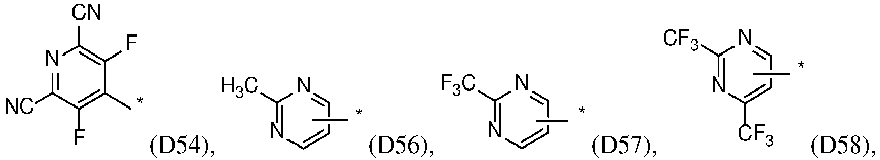 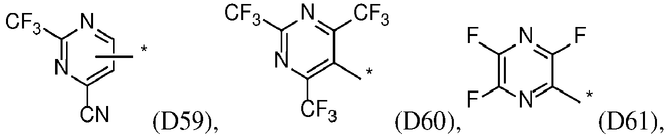 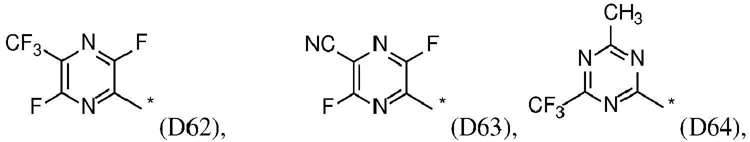 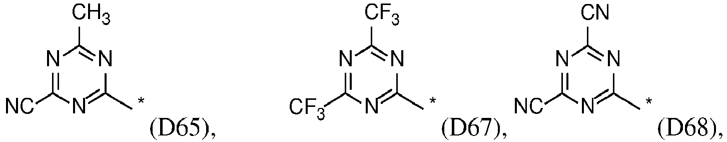 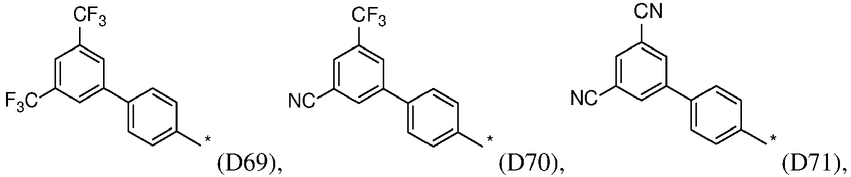 wherein the "^{∗}" denotes the binding position.

According to one embodiment of Formula (I), wherein the Ligand L is selected from the group comprising the following Formulae E1 to E211:

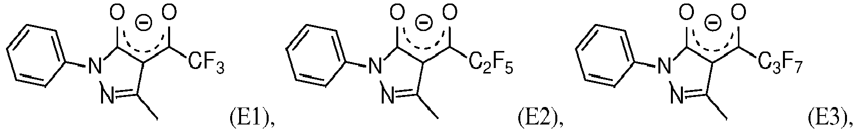

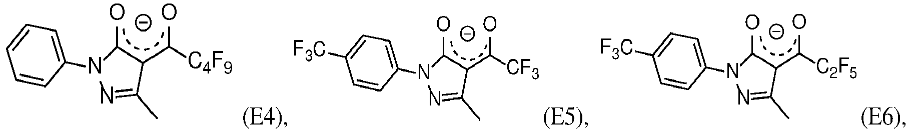

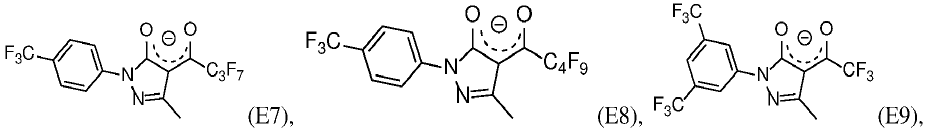

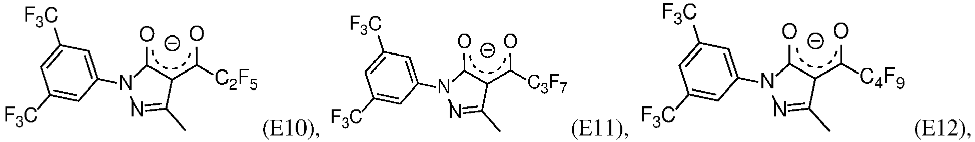

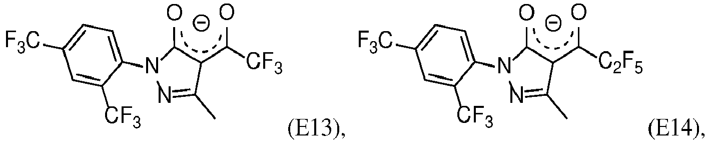

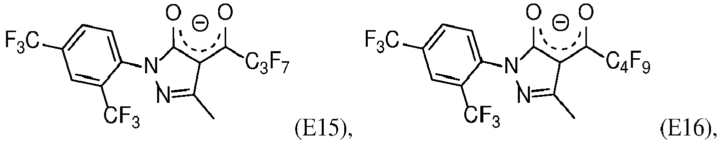

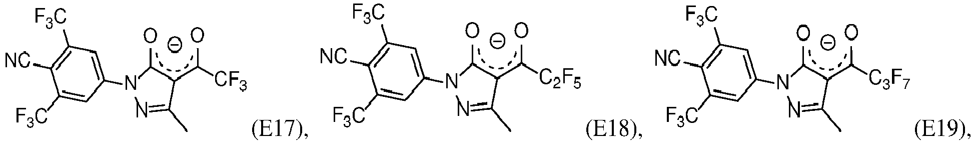

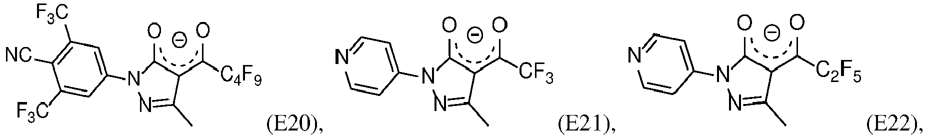

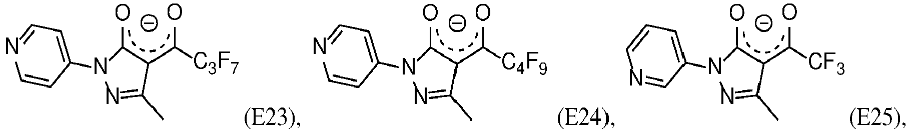

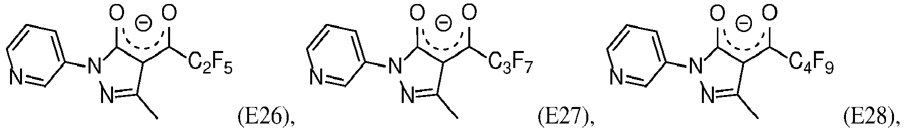

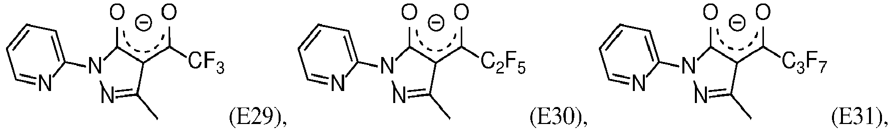

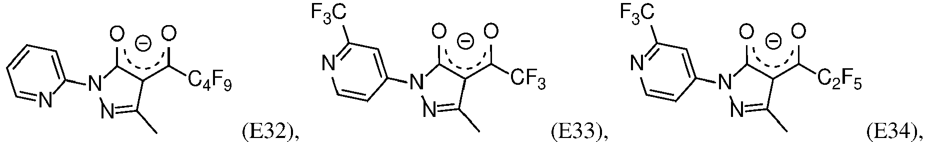

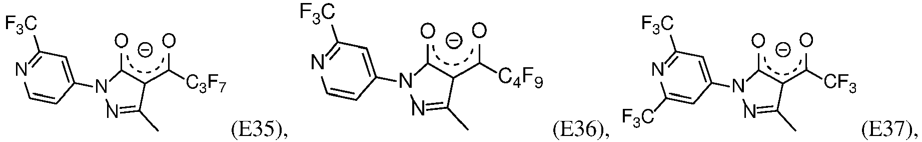

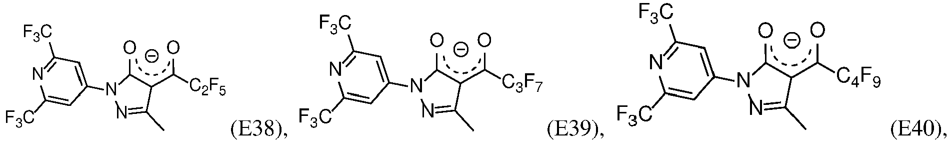

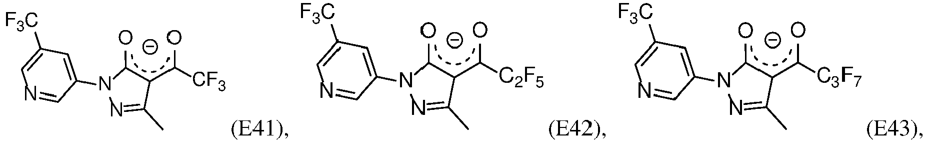

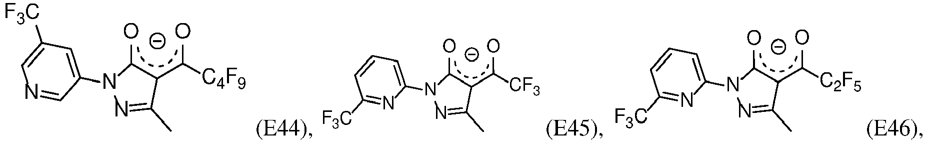

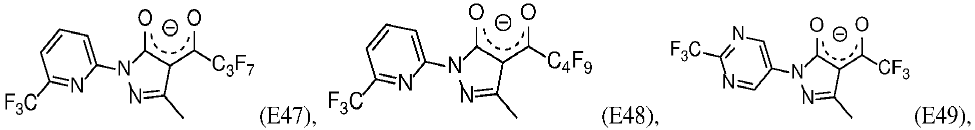

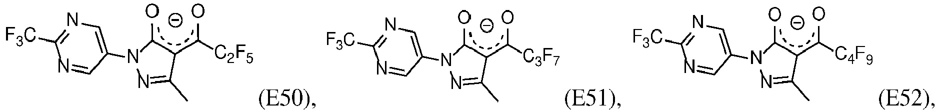

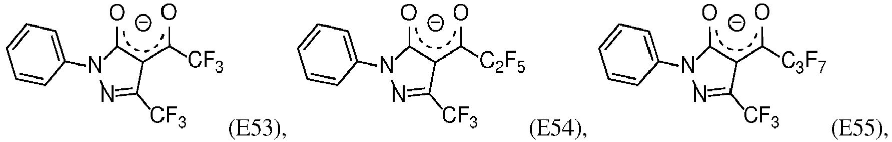

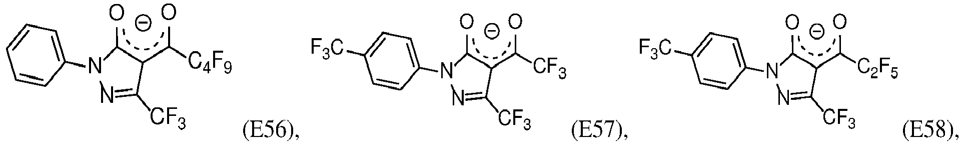

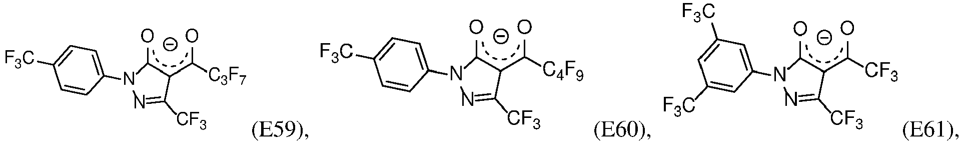

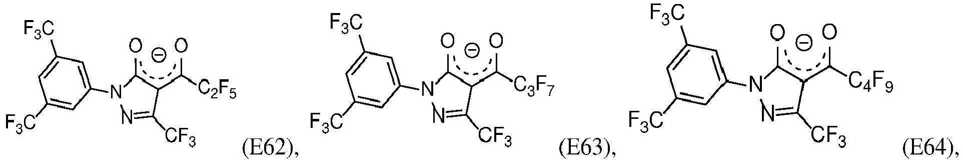

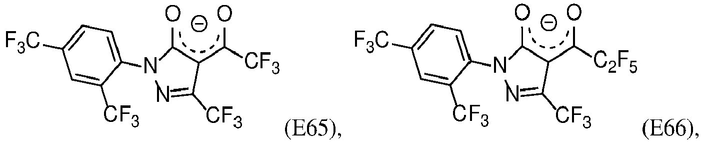

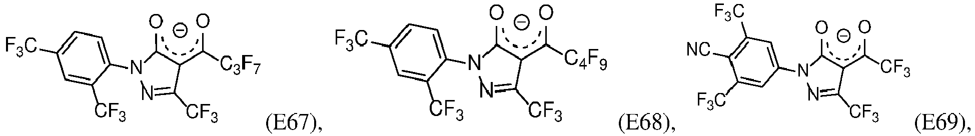

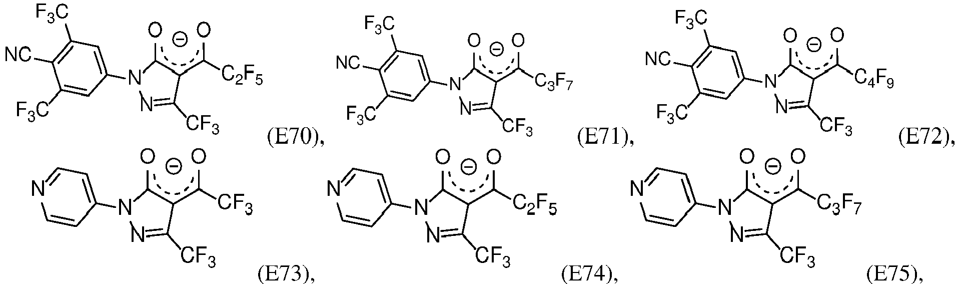

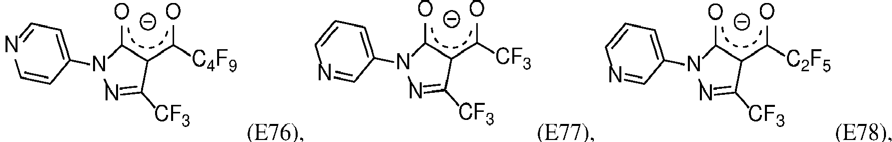

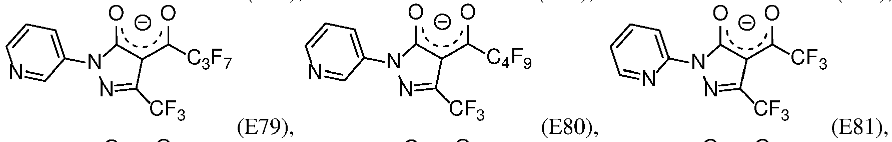

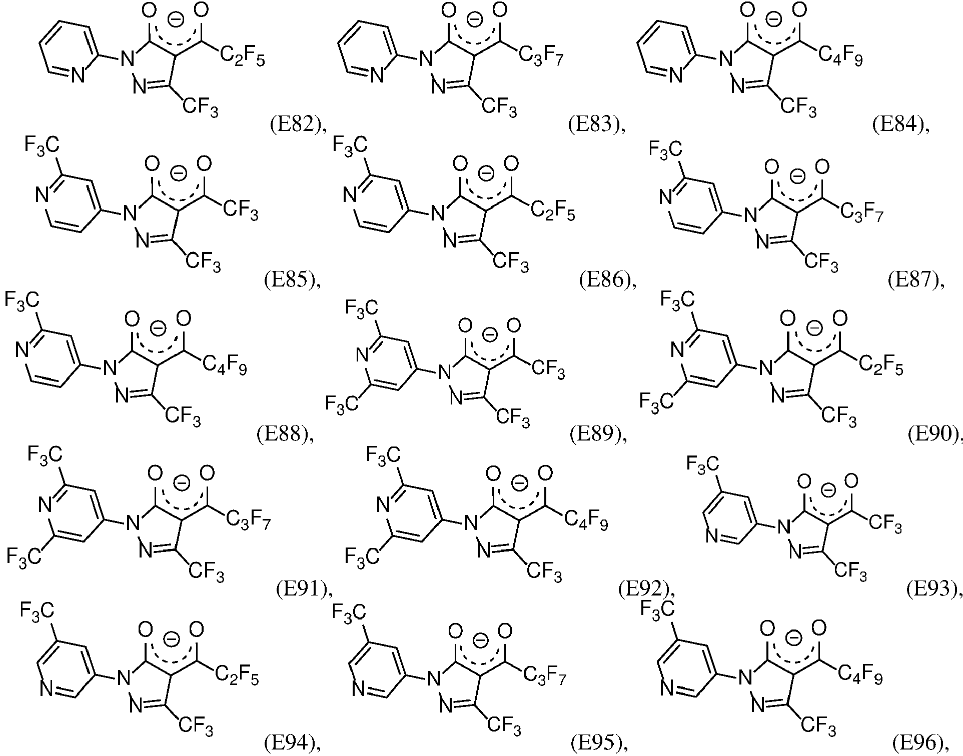

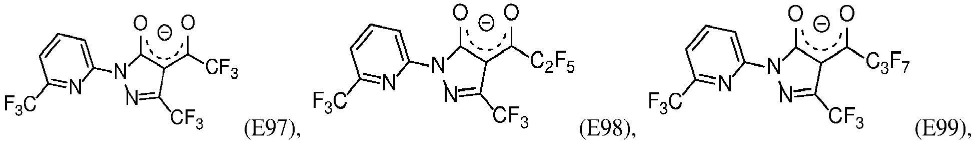

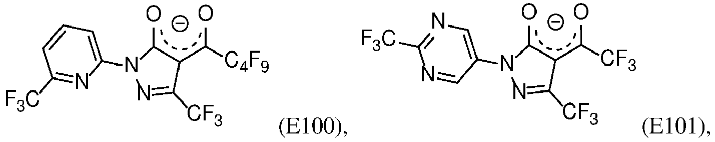

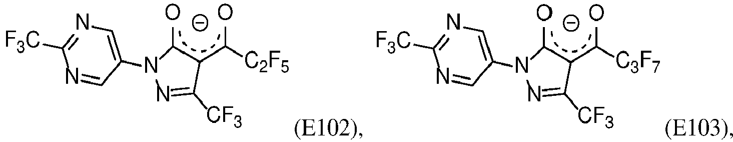

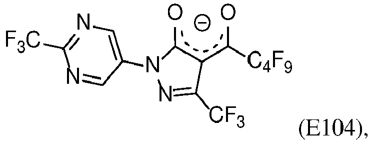

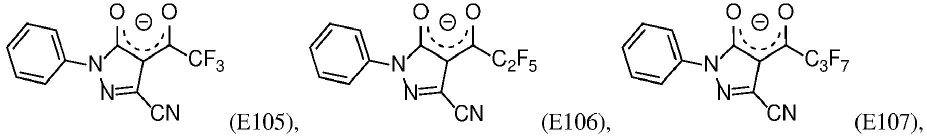

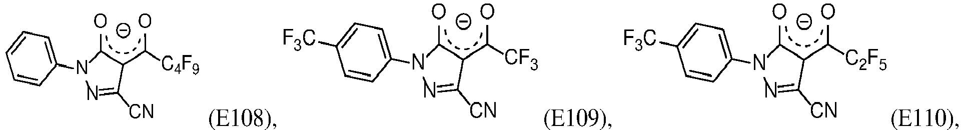

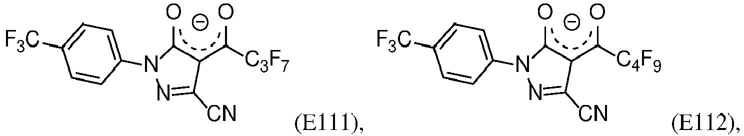

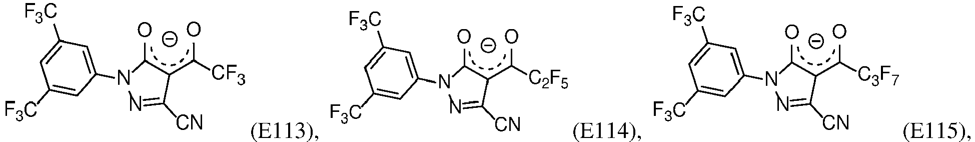

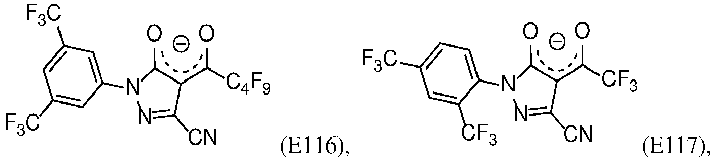

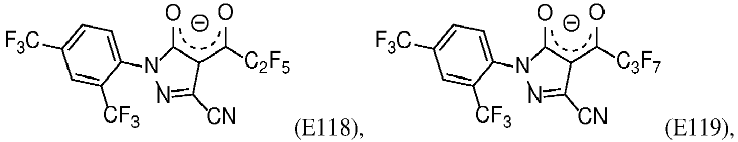

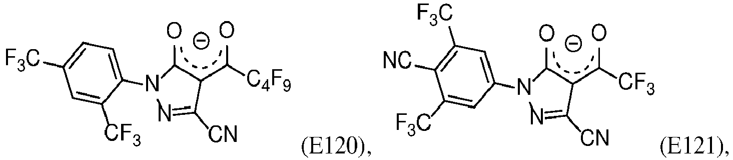

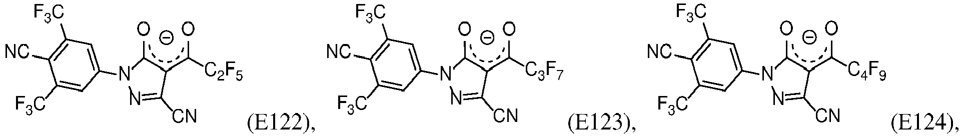

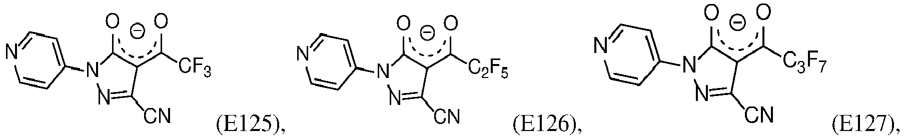

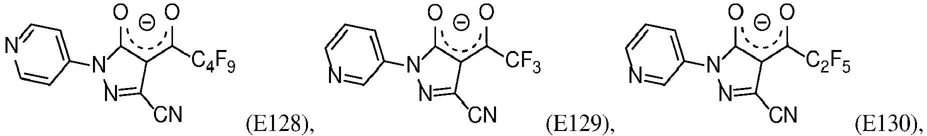

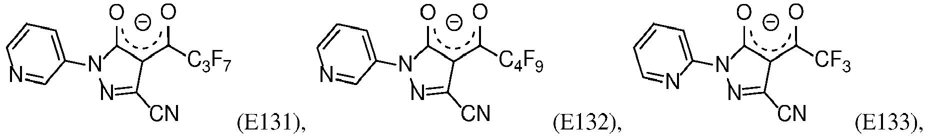

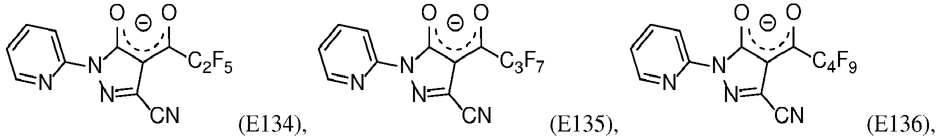

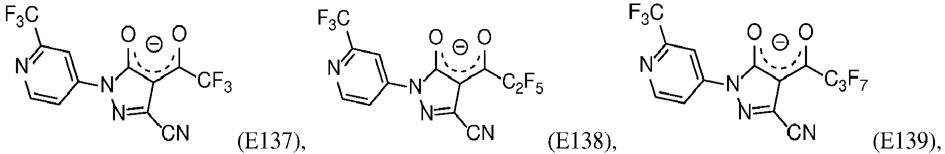

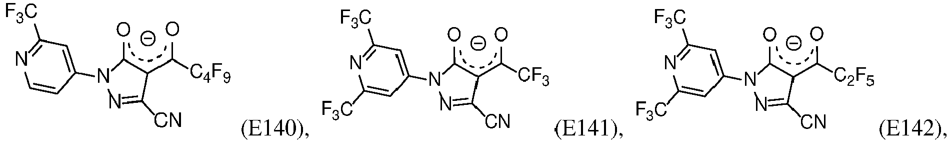

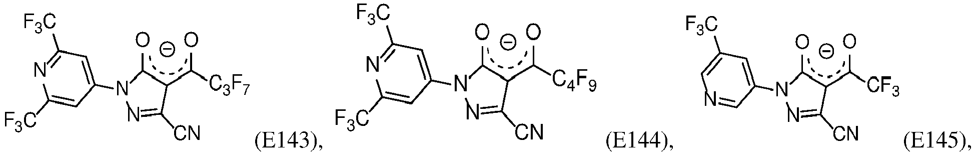

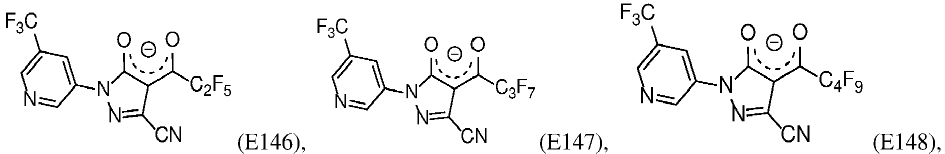

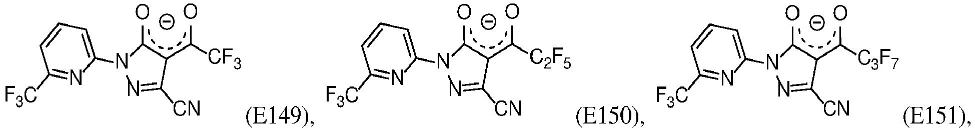

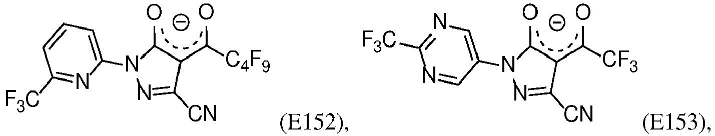

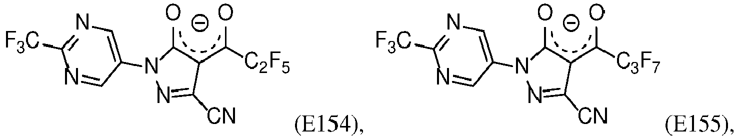

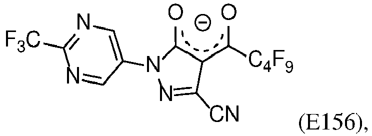

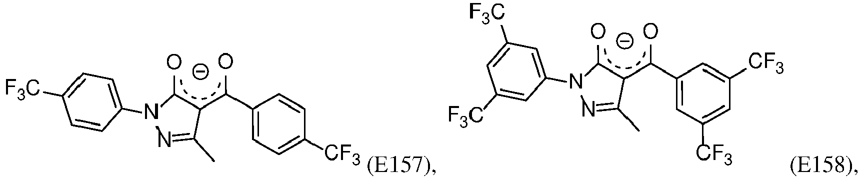

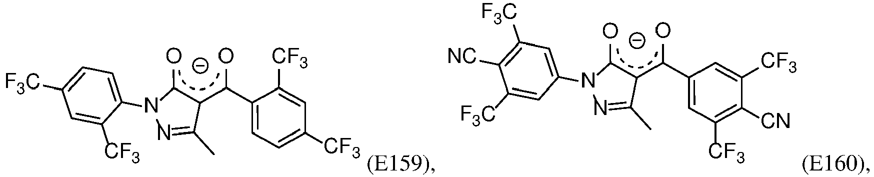

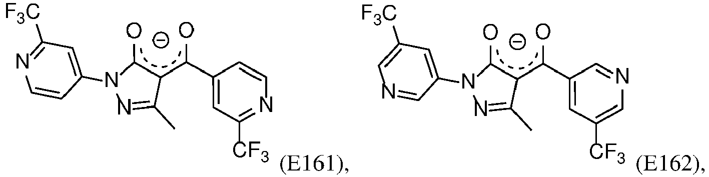

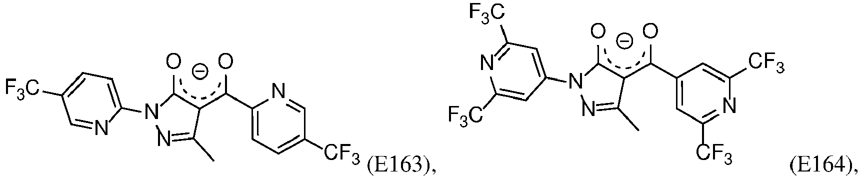

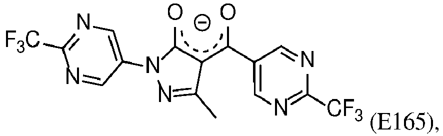

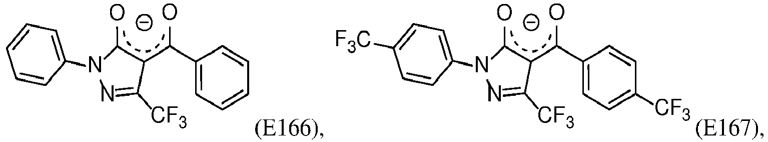

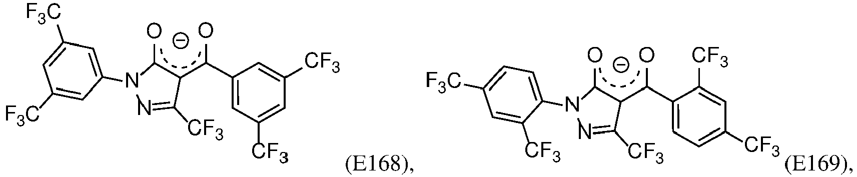

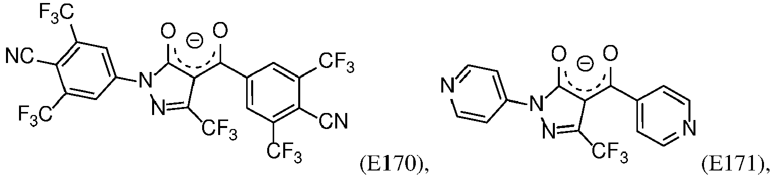

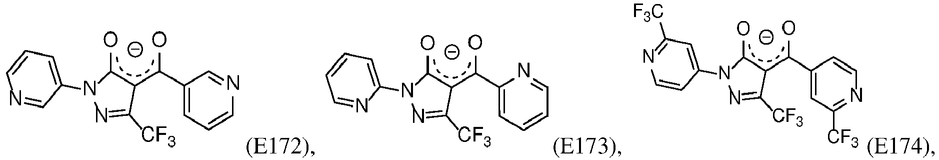

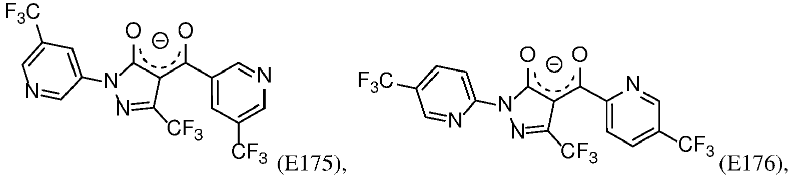

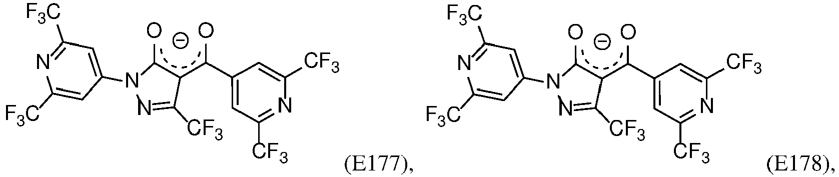

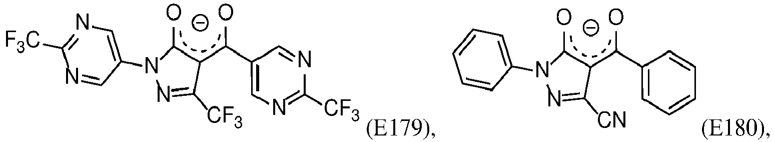

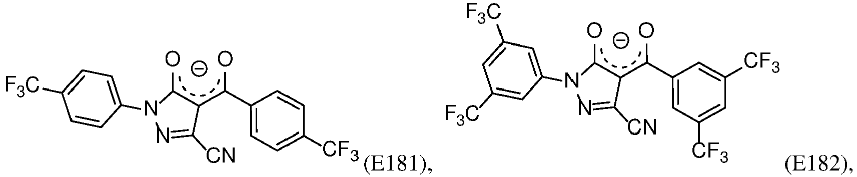

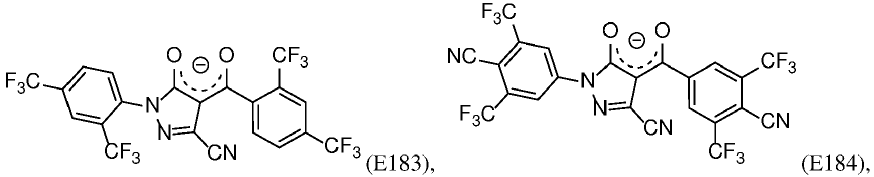

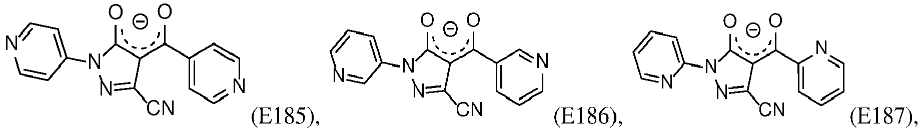

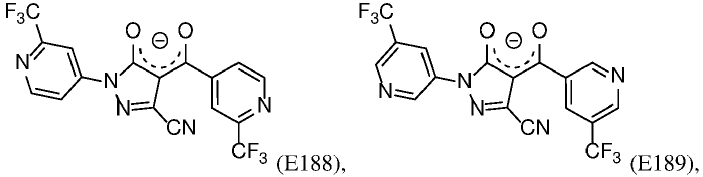

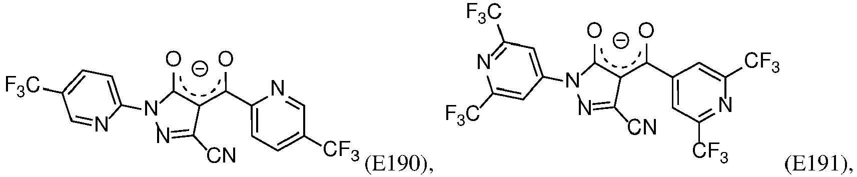

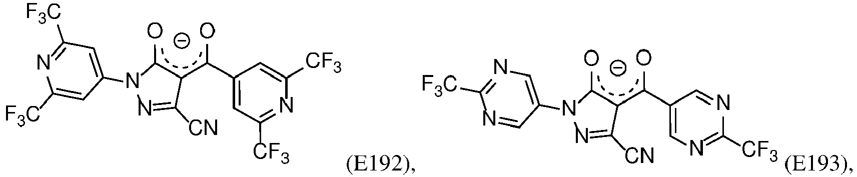

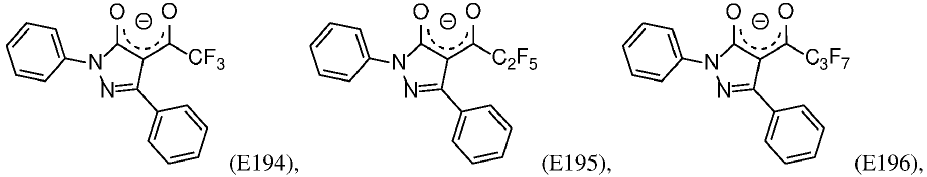

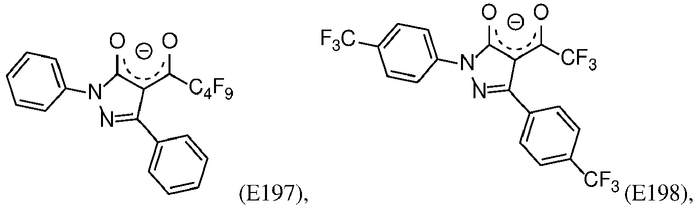

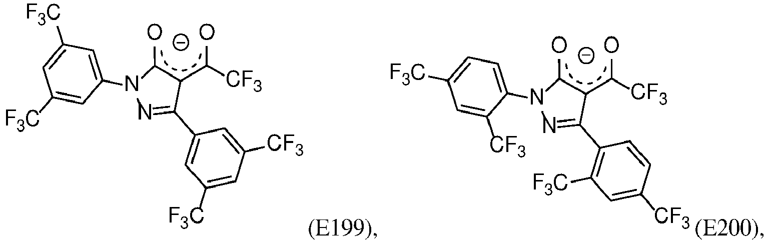

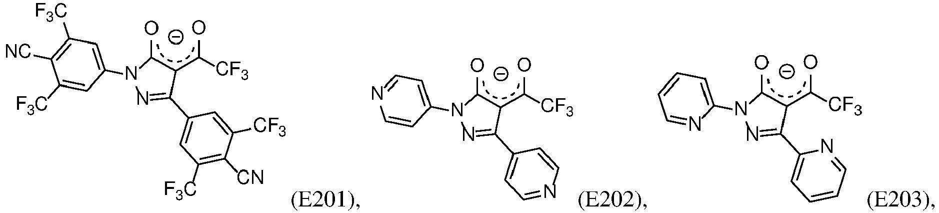

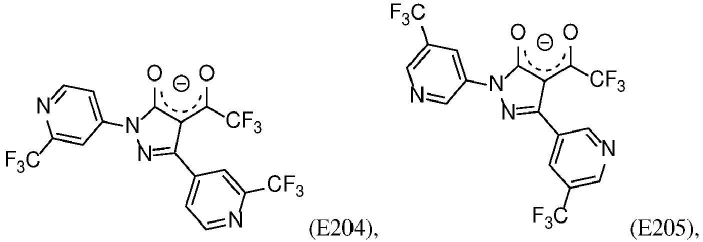

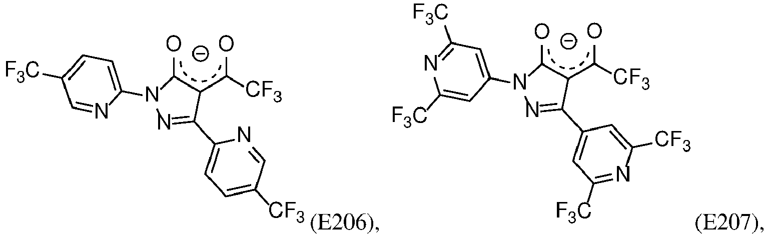

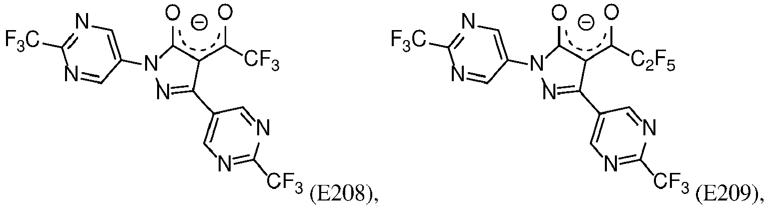

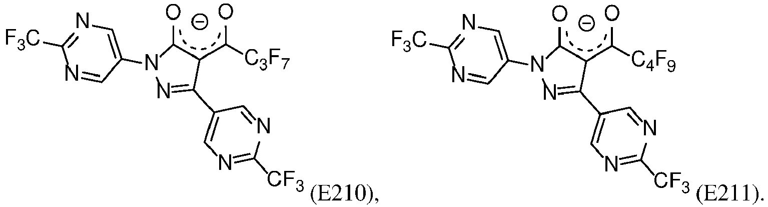

Table 1 shows metal acetylpyrazolonate complexes MC1-MC56 according to Formula (I) that can be suitable used according to the present invention, and comparative compounds CC1-CC5 used in comparative examples

**Table 1**

| Metal acetylpyrazolonate complexes according to Formula (I) | | | | |
|---|---|---|---|---|
| Name | Metal | Structure Ligand | Ligand L | LUMO [eV] (spin) |
| CC1 | Ag(I) | 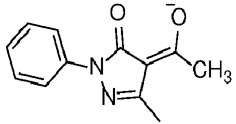 | C1 | -2.07 |
| CC2 | Al(III) | 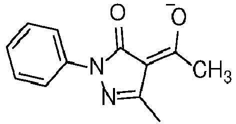 | C1 | -1.92 |
| CC3 | Ag(I) | 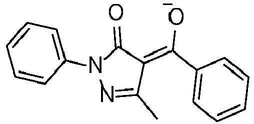 | C2 | -2.11 |
| CC4 | Cu(II) | | | -3.06 (β) |
| | | | 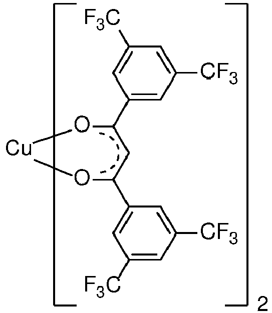 | |
| CC5 | Fe(III) | | 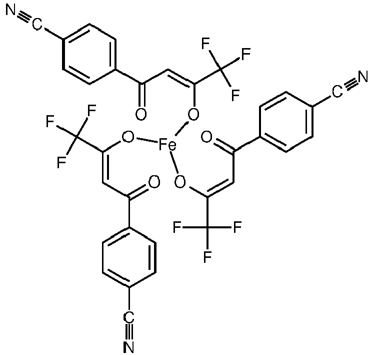 | -4.24 (β) |
| MC1 | Ag(I) | 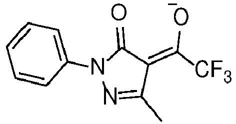 | E1 | -2.20 |
| MC2 | Ir(III) | | E1 | -2.66 |
| | | 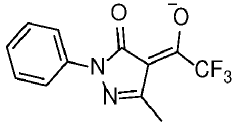 | | |
| MC3 | In(III) | 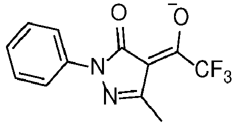 | E1 | -2.67 |
| MC4 | +Al(III) | 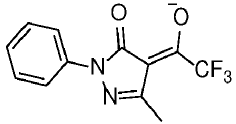 | E1 | -2.80 |
| MC5 | Mn(III) | 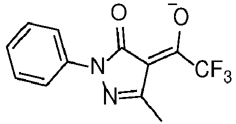 | E1 | -3.34 (β) |
| MC6 | Cu(II) | 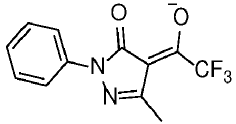 | E1 | -3.61 (β) |
| MC7 | Fe(III) | 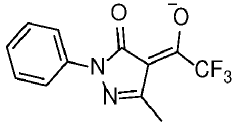 | E1 | -4.25 (β) |
| MC8 | Ru(III) | 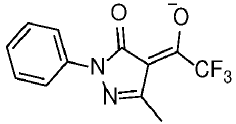 | E1 | -4.37 (β) |
| MC9 | Ce(IV) | 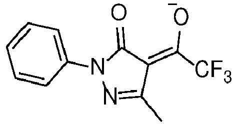 | E1 | -4.50 |
| MC10 | Cu(II) | 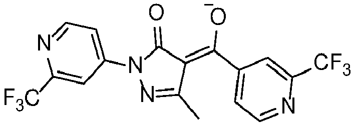 | E161 | -3.58 (β) |
| MC11 | Cu(II) | 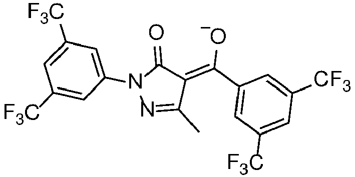 | E158 | -3.50 (β) |
| MC12 | Cu(II) | | E177 | -3.71 (β) |
| | | 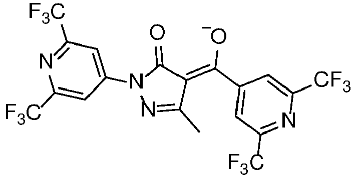 | | |
| MC13 | Cu(II) | 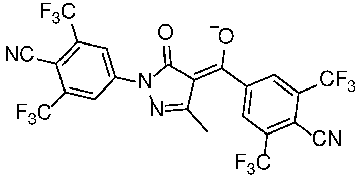 | E160 | -3.74 (β) |
| MC14 | Cu(II) | 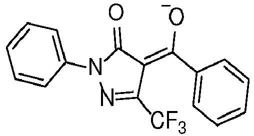 | E53 | -3.46 (β) |
| MC15 | Cu(II) | 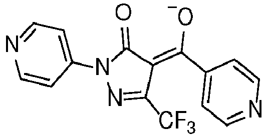 | E171 | -3.68 (β) |
| MC16 | Cu(II) | | E174 | -3.80 (β) |
| | | 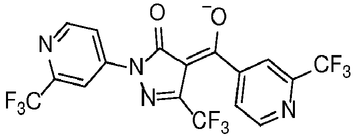 | | |
| MC17 | Cu(II) | 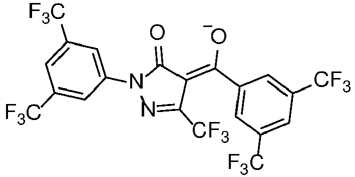 | E168 | -3.75 (β) |
| MC18 | Cu(II) | 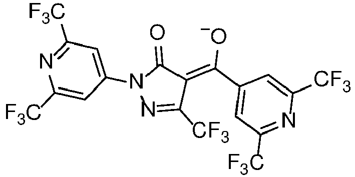 | E177 | -3.93 (β) |
| MC19 | Cu(II) | 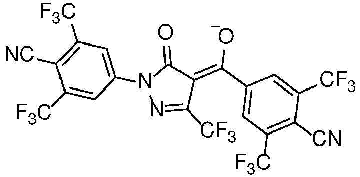 | E170 | -3.95 (β) |
| MC20 | Cu(II) | | E180 | -3.54 (β) |
| | | 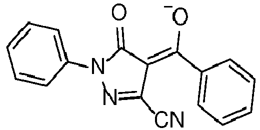 | | |
| MC21 | Cu(II) | 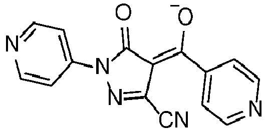 | E185 | -3.77 (β) |
| MC22 | Cu(II) | 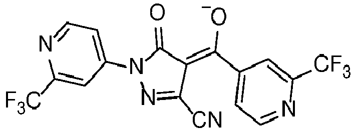 | E188 | -3.88 (β) |
| MC23 | Cu(II) | 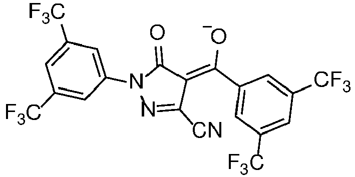 | E182 | -3.84 (β) |
| MC24 | Cu(II) | 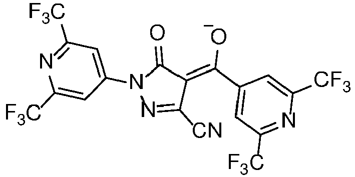 | E191 | -4.02 (β) |
| MC25 | Cu(II) | 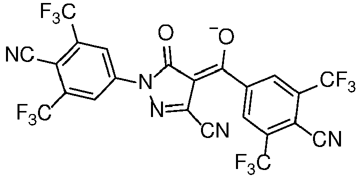 | E184 | -4.02 (β) |
| MC26 | Cu(II) | 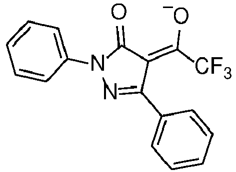 | E194 | -3.66 (β) |
| MC27 | Cu(II) | 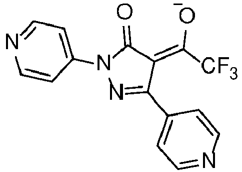 | E202 | -3.84 (β) |
| MC28 | Cu(II) | 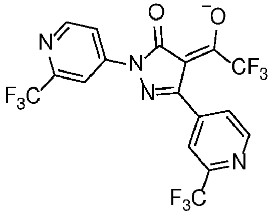 | E204 | -3.94 (β) |
| MC29 | Cu(II) | 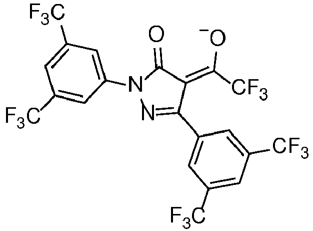 | E199 | -3.87 (β) |
| MC30 | Cu(II) | 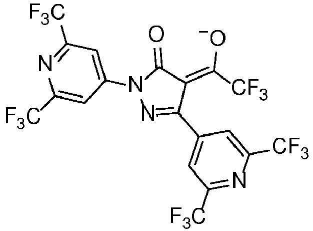 | E207 | -4.05 (β) |
| MC31 | Cu(II) | 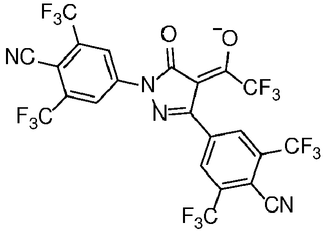 | E201 | -4.06 (β) |
| MC32 | Cu(II) | | E169 | -3.71 (β) |
| | | 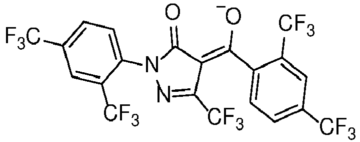 | | |
| MC33 | Fe(III) | 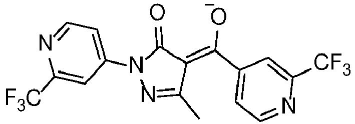 | E161 | -4.20 (β) |
| MC34 | Fe(III) | 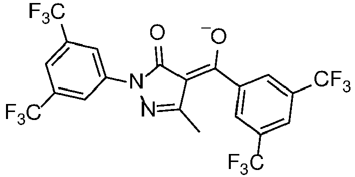 | E158 | -4.11 (β) |
| MC35 | Fe(III) | 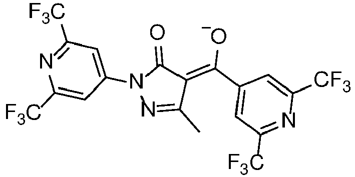 | E164 | -4.38 (β) |
| MC36 | Fe(III) | | E160 | -4.39 (β) |
| | | 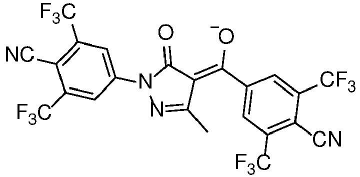 | | |
| MC37 | Fe(III) | 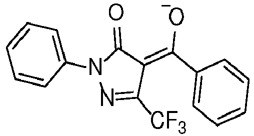 | E166 | -4.02 (β) |
| MC38 | Fe(III) | 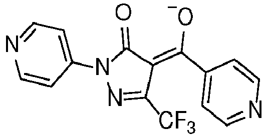 | E171 | -4.33 (β) |
| MC39 | Fe(III) | 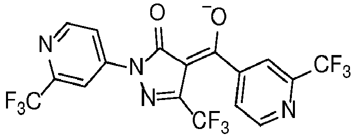 | E174 | -4.50 (β) |
| MC40 | Fe(III) | | E168 | -4.39 (β) |
| | | 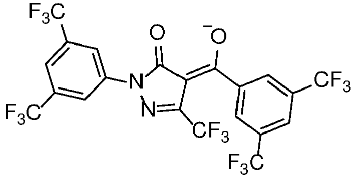 | | |
| MC41 | Fe(III) | 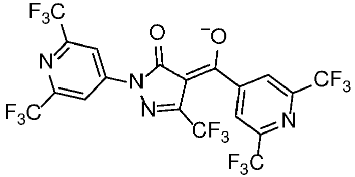 | E178 | -4.67 (β) |
| MC42 | Fe(III) | 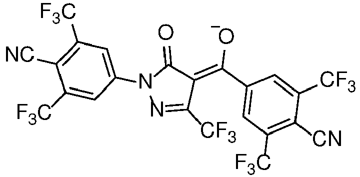 | E170 | -4.66 (β) |
| MC43 | Fe(III) | 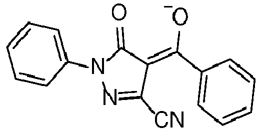 | E180 | -4.10 (β) |
| MC44 | Fe(III) | | E185 | -4.42 (β) |
| | | 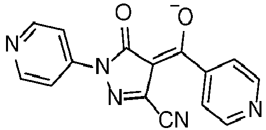 | | |
| MC45 | Fe(III) | 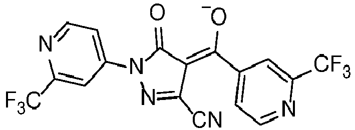 | E188 | -4.60 (β) |
| MC46 | Fe(III) | 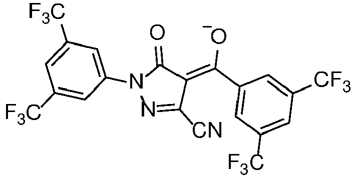 | E182 | -4.49 (β) |
| MC47 | Fe(III) | 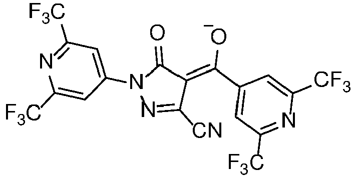 | E191 | -4.77 (β) |
| MC48 | Fe(III) | | E184 | -4.76 (β) |
| | | 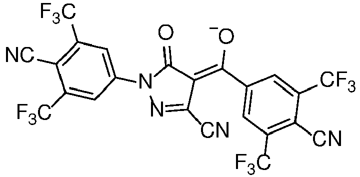 | | |
| MC49 | Fe(III) | 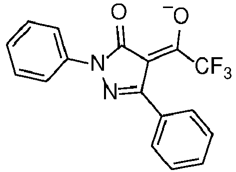 | E194 | -4.28 (β) |
| MC50 | Fe(III) | 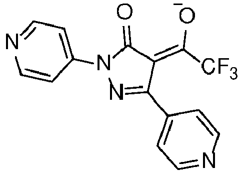 | E202 | -4.53 (β) |
| MC51 | Fe(III) | 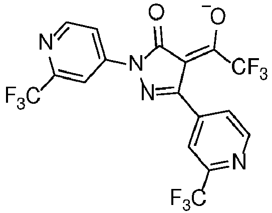 | E204 | -4.67 (β) |
| MC52 | Fe(III) | | E199 | -4.58 (β) |
| | | 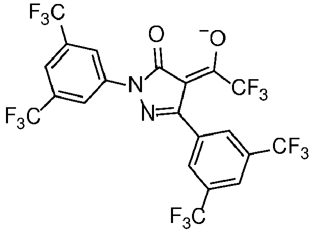 | | |
| MC53 | Fe(III) | 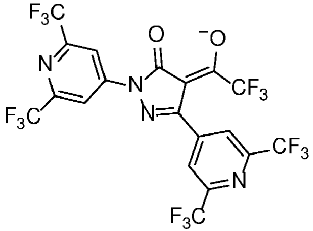 | E207 | -4.81 (β) |
| MC54 | Fe(III) | 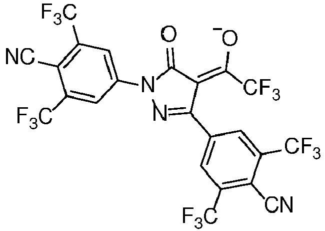 | E201 | -4.81 (β) |
| MC55 | Fe(III) | 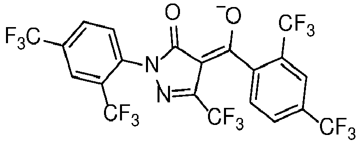 | E168 | -4.11 (β) |
| MC-56 | Cu(II) | 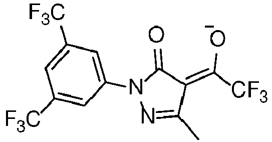 | E9 | -3.76 (β) |

According to one embodiment of Formula (I), wherein the AL is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (III); 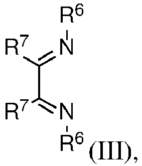 wherein
- R⁶ and R⁷: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### Semiconductor material

According to another aspect, wherein a semiconductor material comprises at least one compound of Formula (I) according to the present invention.

According to one embodiment, wherein the semiconductor material comprises in addition at least one covalent matrix compound or at least one substantially covalent matrix compound.

According to another aspect, wherein a semiconductor material comprises at least one compound of Formula (I) according to the present invention and in addition at least one covalent matrix compound or at least one substantially covalent matrix compound.

### Substantially covalent matrix compound/ covalent matrix compound

The substantially covalent matrix compound, also named matrix compound, may be an organic aromatic matrix compounds, which comprises organic aromatic covalent bonded carbon atoms. The substantially covalent matrix compound may be an organic compound, consisting substantially from covalently bound C, H, O, N, S, which may optionally comprise also covalently bound B, P or Si. The substantially covalent matrix compound may be an organic aromatic covalent bonded compound, which is free of metal atoms, and the majority of its skeletal atoms may be selected from C, O, S, N and preferably from C, O and N, wherein the majority of atoms are C-atoms. Alternatively, the covalent matrix compound is free of metal atoms and majority of its skeletal atoms may be selected from C and N, preferably the covalent matrix compound is free of metal atoms and majority of its skeletal atoms may be selected from C and the minority of its skeletal atoms may be N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment of the present invention, the substantially covalent matrix compound may be free of alkoxy groups.

Preferably, the substantially covalent matrix compound comprises at least one arylamine moiety, alternatively a diarylamine moiety, alternatively a triarylamine moiety.

Preferably, the substantially covalent matrix compound is free of TPD or NPB.

### Compound of formula (IV) or a compound of formula (V)

According to the present invention, the substantially covalent matrix compound or covalent matrix compound, also referred to as matrix compound herein, may comprises at least one arylamine compound, diarylamine compound, triarylamine compound, a compound of formula (IV) or a compound of formula (V): 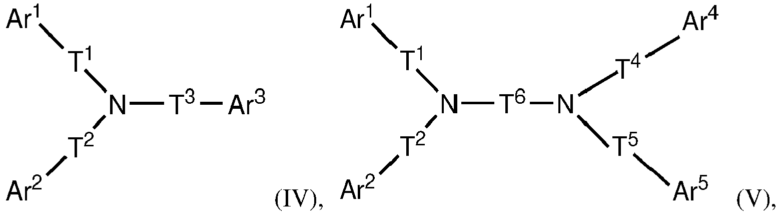 wherein:
- T¹, T², T³, T⁴ and T⁵: are independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment of the electronic device, wherein the substantially covalent matrix compound comprises a compound of formula (IV) or formula (V): 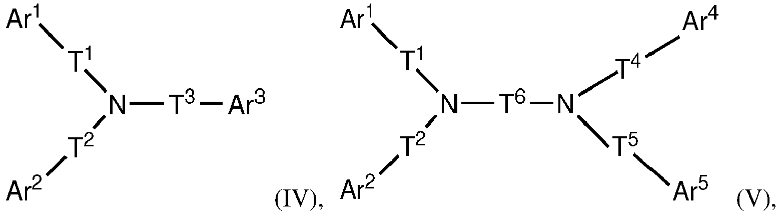 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, C₆ to C₁₈ aryl, C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl.

Thereby, the compound of formula (IV) or (V) may have a rate onset temperature suitable for mass production.

According to an embodiment of the electronic device, wherein the substantially covalent matrix compound comprises a compound of formula (IV) or formula (V): 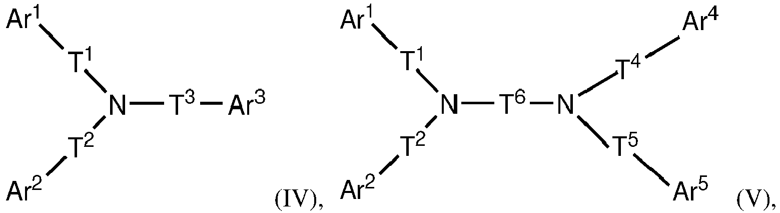 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the electronic device, wherein the substantially covalent matrix compound comprises a compound of formula (IV) or formula (V): 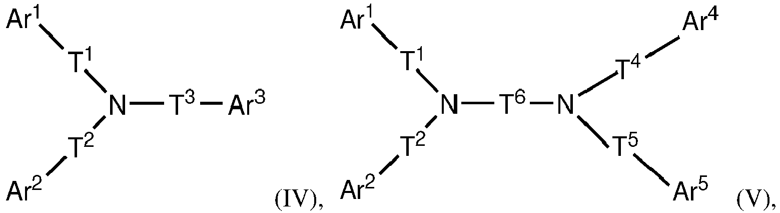 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IV) or (V) may have a rate onset temperature suitable for mass production.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from B1 to B16: 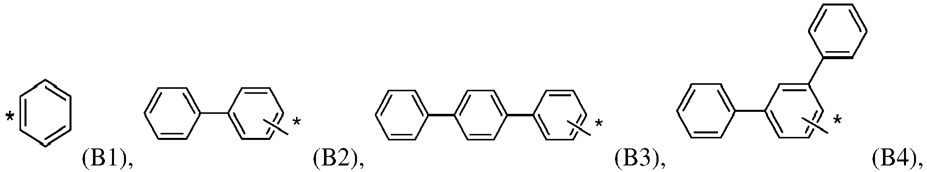 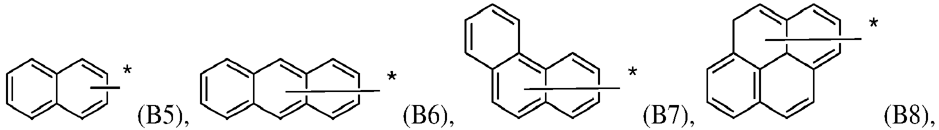 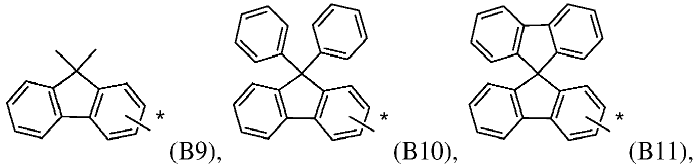 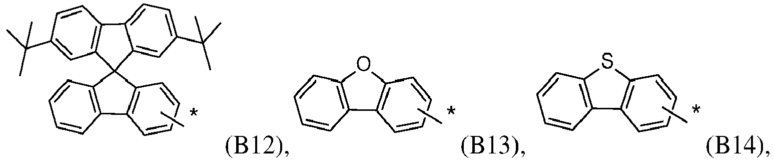 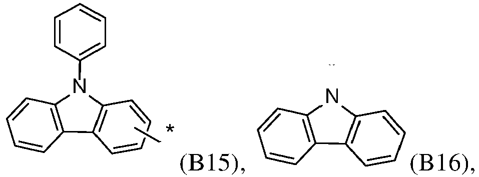 wherein the asterix "^{∗}" denotes the binding position.

According to an embodiment, wherein Ar¹, Ar², Ar³, Ar⁴ and Ar⁵ may be independently selected from B1 to B15; alternatively selected from B1 to B10 and B13 to B15.

According to an embodiment, wherein Ar¹, Ar², Ar³, Ar⁴ and Ar⁵ may be independently selected from the group consisting of B1, B2, B5, B7, B9, B10, B13 to B16.

The rate onset temperature may be in a range particularly suited to mass production, when Ar¹, Ar², Ar³, Ar⁴ and Ar⁵ are selected in this range.

The "matrix compound of formula (IV) or formula (V) " may be also referred to as "hole transport compound".

According to one embodiment the compound of formula (IV) or formula (V) may comprises at least ≥ 1 to ≤ 6 substituted or unsubstituted aromatic fused ring systems comprising heteroaromatic rings.

According to one embodiment the compound of formula (IV) or formula (V) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the compound of formula (IV) or formula (V) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the compound of formula (IV) or formula (V) may comprises at least ≥ 1 to ≤ 3 or 2 substituted or unsubstituted unsaturated 5- to 7-member ring of a heterocycle.

According to one embodiment the compound of formula (IV) or formula (V) may comprises at least ≥ 1 to ≤ 3 or 2 substituted or unsubstituted unsaturated 7-member ring of a heterocycle.

According to one embodiment substituted or unsubstituted aromatic fused ring systems of the compound of formula (IV) or formula (V) may comprises at least ≥ 1 to ≤ 3 or 2 substituted or unsubstituted unsaturated 5- to 7-member ring of a heterocycle.

According to one embodiment the substituted or unsubstituted aromatic fused ring systems of the matrix compound of formula (IV) or formula (V) may comprises at least ≥ 1 to ≤ 3 or 2 substituted or unsubstituted unsaturated 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment the compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system comprises at least ≥ 1 to ≤ 3 or 2 substituted or unsubstituted unsaturated 5- to 7-member ring of a heterocycle.

According to one embodiment the compound of formula (IV) or formula (V) may comprises:
- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic C₆ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
      the hole transport compound or the hole transport compound according to formula (I) comprises at least ≥ 1 to ≤ 4, preferably 2 or 3 aromatic 5-member-rings, preferably hetero aromatic 5-member-rings, and/or
      the hole transport compound or the hole transport compound according to formula (I) comprises at least 1 or 2 unsaturated 5- to 7-member-ring of a heterocycle, preferably at least 1 or 2 unsaturated 7-member-ring of a heterocycle.

According to one embodiment the compound of formula (IV) or formula (V) may comprises a hetero-atom, which may be selected from the group comprising O, S, N, B or P, preferably the hetero-atom may be selected from the group comprising O, S or N.

According to one embodiment the matrix compound of formula (IV) or formula (V) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
   wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.

According to one embodiment the compound of formula (IV) or formula (V) may be free of hetero-atoms which are not part of an aromatic ring and/or part of an unsaturated 7-member-ring, preferably the hole transport compound or the hole transport compound according to formula (I) may be free on N-atoms except N-atoms which are part of an aromatic ring or are part of an unsaturated 7-member-ring.

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the present invention, wherein the compound of formula (IV) or formula (V) are selected from K1 to K16:

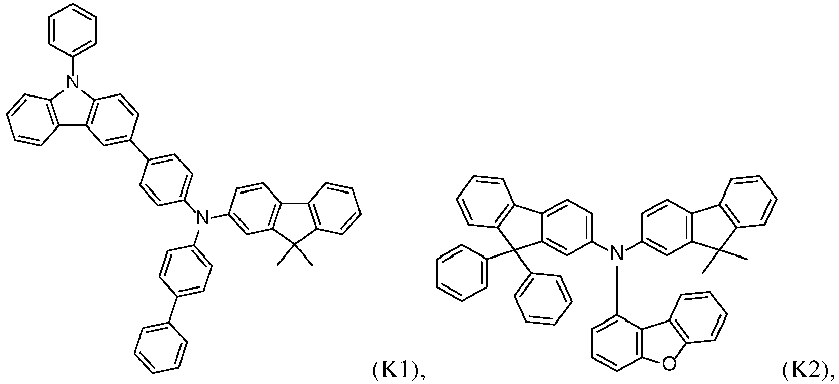

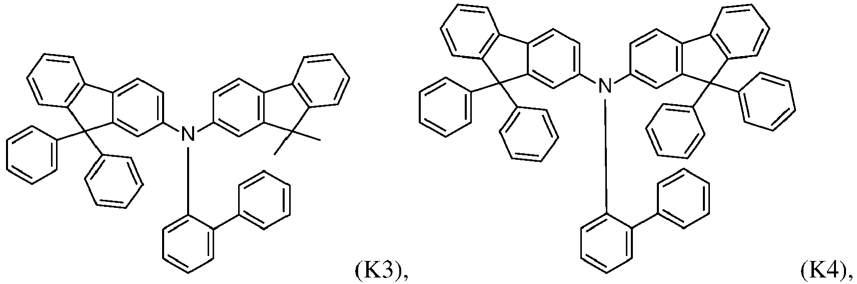

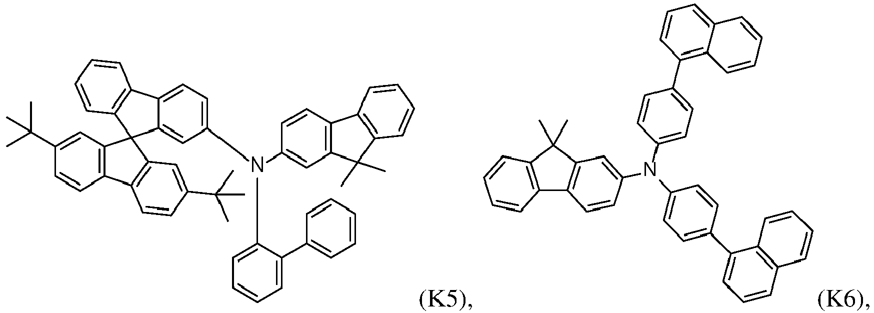

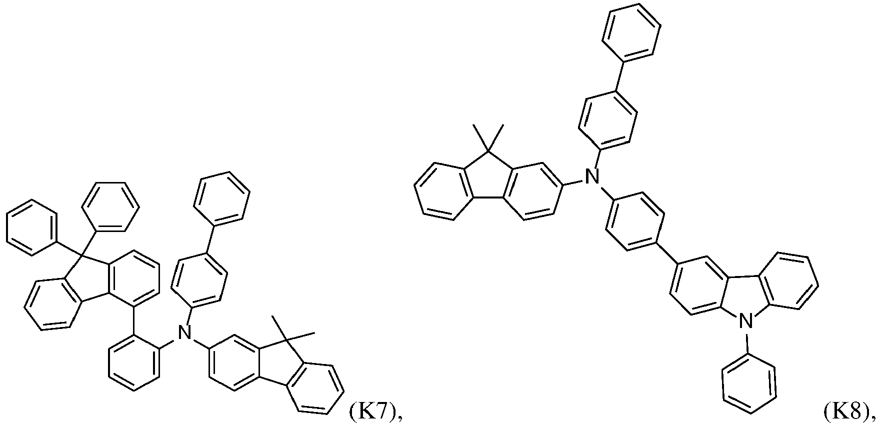

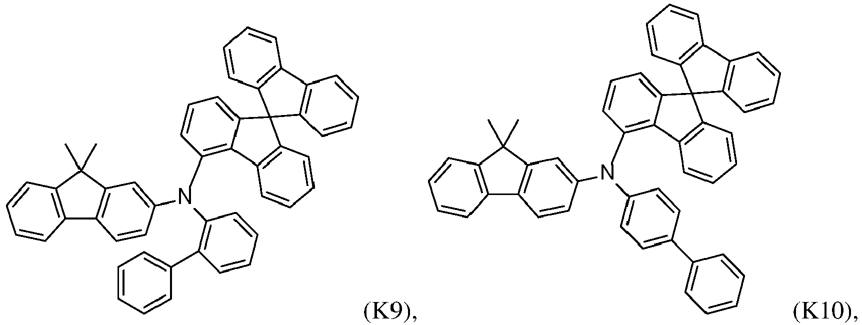

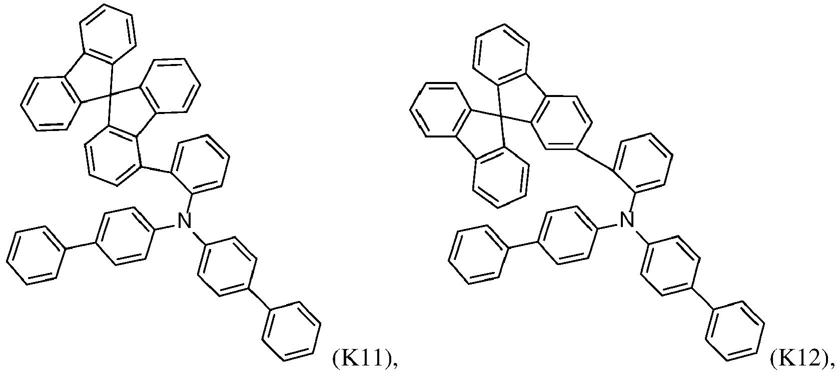

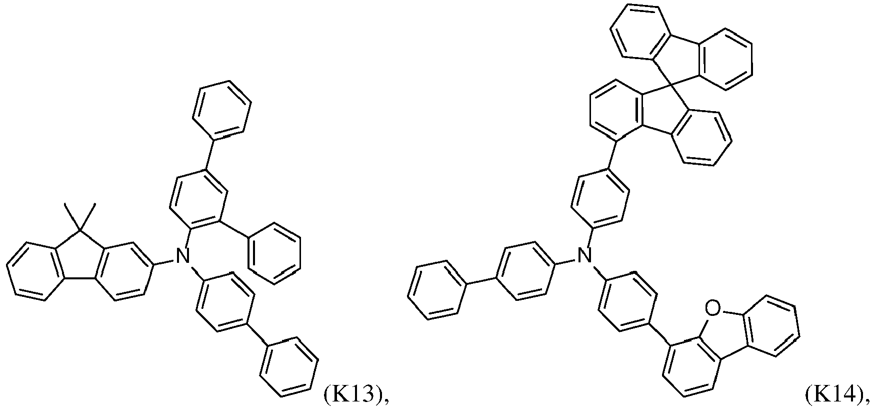

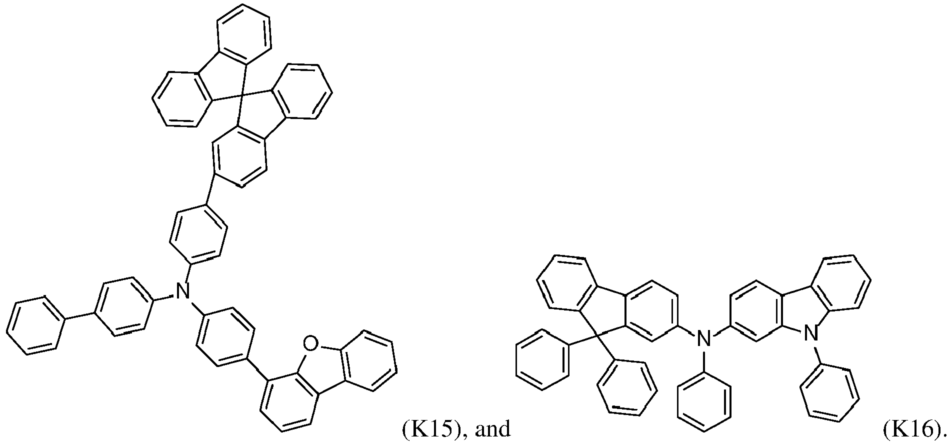

The substantially covalent matrix compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the hole transport compound that is used to form the HIL, and the desired structure and thermal properties of the HIL. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the hole transport compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of a metal complex according to formula (I).

The thickness of the HIL may be in the range from about 1 nm to about 15 nm, and for example, from about 2 nm to about 15 nm, alternatively about 2 nm to about 12 nm.

When the thickness of the HIL is within this range, the HIL may have excellent hole injecting characteristics, without a substantial penalty in driving voltage.

According to one embodiment of the present invention, the hole injection layer may comprise:
- at least about ≥ 0.5 wt.-% to about ≤ 30 wt.-%, preferably about ≥ 0.5 wt.-% to about ≤ 20 wt.- %, and more preferred about ≥ 15 wt.-% to about ≤ 1 wt.-% of a metal complex according to formula (I), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the a metal complex according to formula (I) is lower than the wt.-% of the substantially covalent matrix compound; wherein the weight-% of the components are based on the total weight of the hole injection layer.

Preferably, the hole injection layer may be free of ionic liquids, metal phthalocyanine, CuPc, HAT-CN, Pyrazino[2,3-f][1,10]phenanthroline-2,3-dicarbonitrile, F₄TCNQ, metal fluoride and/or metal oxides, wherein the metal in the metal oxide is selected from Re and/or Mo. Thereby, the hole injection layer may be deposited under conditions suitable for mass production.

According to an embodiment of the electronic device, wherein the hole injection layer is non-emissive.

It is to be understood that the hole injection layer is not part of the anode layer.

### Hole transport layer / Hole injection layer

According to a further embodiment the organic electronic device further comprises a hole transport layer, wherein the hole transport layer is arranged between the hole injection layer and the at least one emission layer.

In one embodiment of the present invention, the hole injection layer comprises a substantially covalent matrix compound.

In one embodiment of the present invention, the hole transport layer comprises a substantially covalent matrix compound.

In one embodiment of the present invention, the hole transport layer comprises a substantially covalent matrix compound, wherein the substantially covalent matrix compound in the hole injection layer and hole transport layer are selected the same.

Preferably, the matrix compound of the hole injection layer is free of metals and/or ionic bonds.

### Further layers

In accordance with the invention, the electronic device may comprise, besides the layers already mentioned above, further layers. Exemplary embodiments of respective layers are described in the following:

### Substrate

The substrate may be any substrate that is commonly used in manufacturing of electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a transistor backplane. Preferably, the substrate is a silicon substrate or transistor backplane.

### Anode layer

The anode layer, also named anode electrode, may be formed by depositing or sputtering a material that is used to form the anode layer. The material used to form the anode layer may be a high work-function material, so as to facilitate hole injection. The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

The anode layer may comprise two or more anode sub-layers.

According to one embodiment, the anode layer comprises a first anode sub-layer and a second anode sub-layer, wherein the first anode sub-layer is arranged closer to the substrate and the second anode sub-layer is arranged closer to the cathode layer.

According to one embodiment, the anode layer may comprise a first anode sub-layer comprising or consisting of Ag or Au and a second anode-sub-layer comprising or consisting of transparent conductive oxide.

According to one embodiment, the anode layer comprises a first anode sub-layer, a second anode sub-layer and a third anode sub-layer, wherein the first anode sub-layer is arranged closer to the substrate and the second anode sub-layer is arranged closer to the cathode layer, and the third anode sub-layer is arranged between the substrate and the first anode sub-layer.

According to one embodiment, the anode layer may comprise a first anode sub-layer comprising or consisting of Ag or Au, a second anode-sub-layer comprising or consisting of transparent conductive oxide and optionally a third anode sub-layer comprising or consisting of transparent conductive oxide. Preferably the first anode sub-layer may comprise or consists of Ag, the second anode-sublayer may comprise or consists of ITO or IZO and the third anode sub-layer may comprises or consists of ITO or IZO.

Preferably the first anode sub-layer may comprise or consists of Ag, the second anode-sublayer may comprise or consists of ITO and the third anode sub-layer may comprise or consist of ITO.

Preferably, the transparent conductive oxide in the second and third anode sub-layer may be selected the same.

According to one embodiment, the anode layer may comprise a first anode sub-layer comprising Ag or Au having a thickness of 100 to 150 nm, a second anode sub-layer comprising or consisting of a transparent conductive oxide having a thickness of 3 to 20 nm and a third anode sub-layer comprising or consisting of a transparent conductive oxide having a thickness of 3 to 20 nm.

### Hole transport layer

According to one embodiment of the electronic device, wherein the electronic device further comprises a hole transport layer, wherein the hole transport layer is arranged between the hole injection layer and the at least one first emission layer.

The hole transport layer may comprise a substantially covalent matrix compound. According to one embodiment the substantially covalent matrix compound of the hole transport layer may be selected from at least one organic compound. The substantially covalent matrix may consist substantially from covalently bound C, H, O, N, S, which optionally comprise in addition covalently bound B, P, As and/or Se.

According to one embodiment of the electronic device, the hole transport layer comprises a substantially covalent matrix compound, wherein the substantially covalent matrix compound of the hole transport layer may be selected from organic compounds consisting substantially from covalently bound C, H, O, N, S, which optionally comprise in addition covalently bound B, P, As and/or Se.

According to one embodiment, the substantially covalent matrix compound of the hole transport layer may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound of the hole injection layer and the substantially covalent matrix compound of the hole transport layer are selected the same.

According to one embodiment of the electronic device, wherein the hole transport layer of the electronic device comprises a substantially covalent matrix compound, preferably the substantially covalent matrix compound in the hole injection layer and hole transport layer are selected the same.

The hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the hole transport compound that is used to form the HTL.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 200 nm, further about 100 nm to about 180 nm, further about 110 nm to about 140 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer may be achieved. The triplet control layer may be selected from triarylamine compounds with a triplet level above the triplet level of the phosphorescent emitter in the adjacent emission layer.

The thickness of the electron blocking layer may be selected between 2 and 20 nm.

### Photoactive layer (PAL)

The photoactive layer converts an electrical current into photons or photons into an electrical current. The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL. It may be provided that the photoactive layer does not comprise the a metal complex according to formula (I). The photoactive layer may be a light-emitting layer or a light-absorbing layer.

### Emission layer (EML)

The at least one first emission layer (EML), also referred to as first emission layer may be formed on the HTL or EBL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to the present invention it is preferred that the electronic device comprises one emission layer that is named "first emission layer". However, the electronic device optionally comprises two emission layers, wherein the first layer is named first emission layer and second layer is named second emission layer.

It may be provided that the at least one emission layer also referred to as first emission layer is free of the matrix compound of the hole injection layer.

It may be provided that the at least one emission layer does not comprise the a metal complex according to formula (I).

The at least one emission layer (EML) may be formed of a combination of a host and an emitter dopant. Example of the host are Alq3, 4,4'-N,N'-dicarbazole-biphenyl (HTC-10), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters; however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)₂Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the at least one emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent emitter dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form an HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitate injection of electrons from the cathode, may be formed on the ETL, preferably directly on the electron transport layer. Examples of materials for forming the EIL include lithium 8-hydroxyquinolinolate (LiQ), LiF, NaCl, CsF, Li₂O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode layer

The cathode layer is formed on the ETL or optional EIL. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer or the electron transport layer.

### Organic electronic device

According to another aspect of the present invention an organic electronic device is provided, wherein the organic electronic device comprising a compound of Formula (I).

According to one embodiment of the present invention, the organic electronic device is selected from the group comprising a light emitting device, thin film transistor, a battery, a display device or a photovoltaic cell, and preferably a light emitting device, preferably the electronic device is part of a display device or lighting device.

According to one embodiment the organic electronic device comprises a semiconductor material, wherein at least one semiconductor material comprises a metal complex of Formula (I).

According to one embodiment the organic electronic device comprising a metal complex according to Formula (I) of the present invention is a light emitting device, a thin film transistor, a battery, a display device or a photovoltaic device, and preferably a light emitting device, preferably the electronic device is part of a display device or lighting device.

According to one embodiment the organic electronic device comprises a semiconductor material, wherein at least one semiconductor material comprises a metal complex of Formula (I), and wherein the organic electronic device is a light emitting device, a thin film transistor, a battery, a display device or a photovoltaic device, and preferably a light emitting device, preferably the electronic device is part of a display device or lighting device.

According to one embodiment, wherein the organic electronic device comprising a first electrode layer, a second electrode layer, at least one photoactive layer and a semiconductor layer, wherein the semiconductor layer is arranged between the first electrode layer and the at least one photoactive layer, the semiconductor layer comprises a metal complex of formula (I)

M^{n_{⊕}}(L^{Θ})n(AL)m (I),

wherein
M is a metal ion;
n is the valency of M and selected from 1 to 4;
L has formula (II) 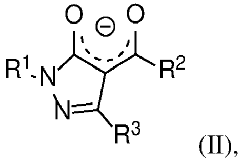
   - R¹ and R²: are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
   - R³: is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
   wherein
   at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
   at least one of R¹, R² and R³ or the group of R¹, R² and R³ comprises at least three atoms selected from the group consisting of halogen, Cl, F or N;
   - AL: is an ancillary ligand which coordinates to the metal M;
   - m: is an integer selected from 0 to 2.

Further embodiments of the metal complex of formula (I) are described throughout the present description to which references are fully relayed too.

### Method of manufacturing

According to another aspect, there is provided a method of manufacturing an electronic device, the method using:
- at least one deposition source, preferably two deposition sources and more preferred at least three deposition sources.

The methods for deposition that may be suitable comprise:
- deposition via vacuum thermal evaporation;
- deposition via solution processing, preferably the processing may be selected from spin-coating, printing, casting; and/or
- slot-die coating.

According to various embodiments, there is provided a method using:
- a first deposition source to release the matrix compound, and
- a second deposition source to release the a metal complex according to formula (I), also named metal complex.

The method comprising the steps of forming the hole injection layer; whereby for an electronic device:
- the hole injection layer is formed by releasing the matrix compound according to the invention from the first deposition source and the a metal complex according to formula (I), also named metal complex, from the second deposition source.

### Ink formulation

According to another aspect of the present invention an ink formulation is provided, wherein the ink formulation comprises at least one compound of Formula (I).

According to one embodiment the ink formulation comprises a solvent. The solvent of the ink formulation may be selected from anisole and/or benzonitrile, preferably it is a mixture of anisole and benzonitrile.

According to one embodiment the mixture of anisole and benzonitrile is of 5 : 1 to 1 : 5, 4 : 1 to 1 : 4, 3 : 1 to 1 : 3, 2 : 1 to 1 : 2 or 1:1; preferred is a mixture of anisole and benzonitrile of 5:1.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiment according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.
FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.
FIG. 4 is a schematic sectional view of an organic light-emitting diode (OLED), according to an exemplary embodiment of the present invention;
FIG. 5 is a schematic sectional view of an organic light-emitting diode (OLED), according to an exemplary embodiment of the present invention.
FIG. 6 is a schematic sectional view of an organic light-emitting diode (OLED), according to an exemplary embodiment of the present invention.

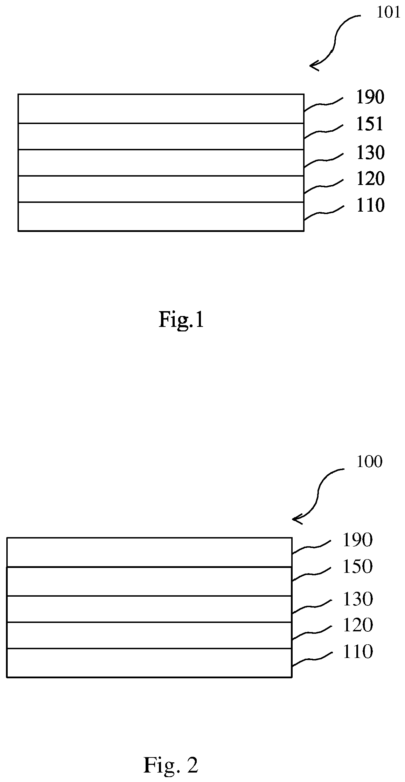

FIG. 1 is a schematic sectional view of an organic electronic device 101, according to an exemplary embodiment of the present invention. The organic electronic device 101 includes a substrate (110), an anode layer (120), a hole injection layer comprising a metal complex according to formula (I) (130), a photoactive layer (PAL) (151) and a cathode layer (190).

FIG. 2 is a schematic sectional view of an organic light-emitting diode (OLED) 100, according to an exemplary embodiment of the present invention. The OLED 100 includes a substrate (110), an anode layer (120), a hole injection layer comprising a metal complex according to formula (I) (130), an emission layer (EML) (150) and a cathode layer (190).

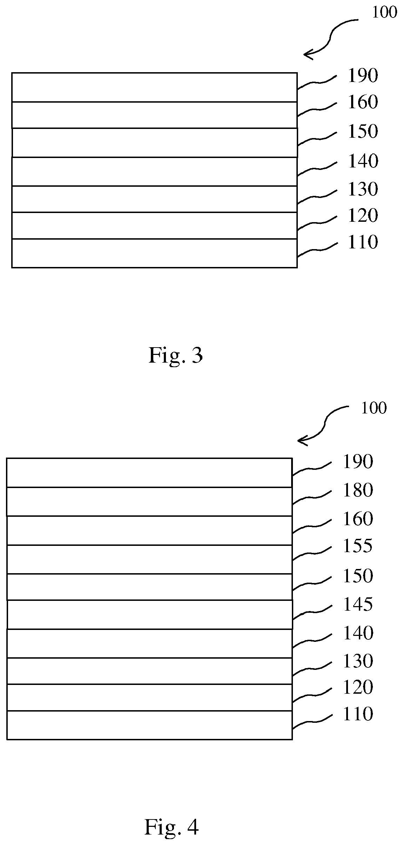

FIG. 3 is a schematic sectional view of an organic light-emitting diode (OLED) 100, according to an exemplary embodiment of the present invention. The OLED 100 includes a substrate (110), an anode layer (120), a hole injection layer comprising a metal complex according to formula (I) (130), a hole transport layer (HTL) (140), an emission layer (EML) (150), an electron transport layer (ETL) (160) and a cathode layer (190).

FIG. 4 is a schematic sectional view of an organic light-emitting diode (OLED) 100, according to an exemplary embodiment of the present invention. The OLED 100 includes a substrate (110), an anode layer (120), a hole injection layer comprising a metal complex according to formula (I) (130), a hole transport layer (HTL) (140), an electron blocking layer (EBL) (145), an emission layer (EML) (150), a hole blocking layer (HBL) (155), an electron transport layer (ETL) (160), an optional electron injection layer (EIL) (180), and a cathode layer (190).

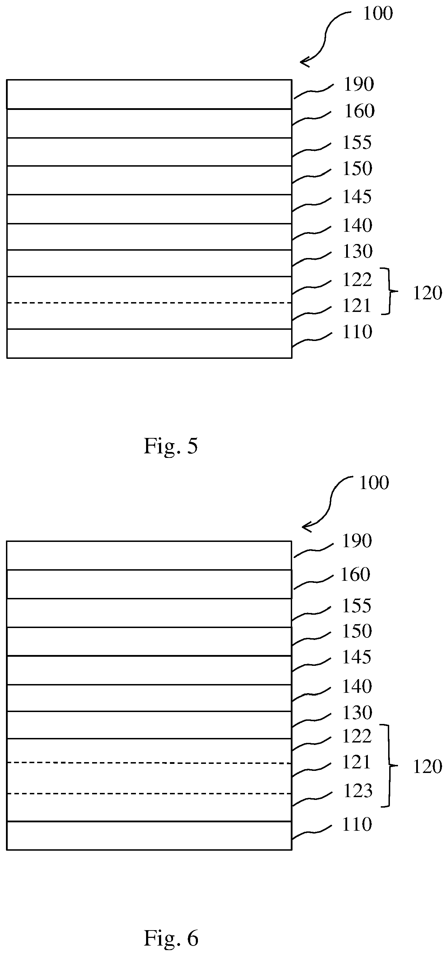

FIG. 5 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), a hole injection layer comprising a metal complex according to formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121), a second anode sub-layer (122) and a third anode sub-layer (123), a hole injection layer comprising a metal complex according to formula (I) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

In the description above the method of manufacture an organic electronic device 101 of the present invention is for example started with a substrate (110) onto which an anode layer (120) is formed, on the anode layer (120), a hole injection layer comprising a metal complex according to formula (I) (130), a photoactive layer (151) and a cathode electrode 190 are formed, exactly in that order or exactly the other way around.

In the description above the method of manufacture an OLED of the present invention is started with a substrate (110) onto which an anode layer (120) is formed, on the anode layer (120), a hole injection layer comprising a metal complex according to formula (I) (130), optional a hole transport layer (140), optional an electron blocking layer (145), an emission layer (150), optional a hole blocking layer (155), optional an electron transport layer (160), optional an electron injection layer (180), and a cathode electrode 190 are formed, exactly in that order or exactly the other way around.

The semiconductor layer comprising a metal complex according to formula (I) (130) can be a hole injection layer.

While not shown in Fig. 1, Fig. 2, Fig. 3, Fig. 4, Fig. 5 and Fig. 6, a capping layer and/or a sealing layer may further be formed on the cathode electrodes 190, in order to seal the OLEDs 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### General method for preparation of the ink formulation

To prepare the ink formulation, the compounds were weighed into vials. Then, the solvent was added. The mixture was stirred for 10 min. The inks were transferred to inert atmosphere. An aliquot of benzonitrile solutions was added to the anisole solution to obtain a solution with a ratio of 5:1 of anisole to benzonitrile solution. The resulting solution was stirred again for at least 10 min at room temperature. The resulting ink formulation had a solid content of 4 wt.-%.

### Dipole moment

The dipole moment |*̅µ̅*̅| of a molecule containing N atoms is given by: $\vec{μ} = {\sum }_{i}^{N} {q}_{i} {\vec{r}}_{ι}$ $\left|\vec{μ}\right| = \sqrt{{μ}_{x}^{2} + {μ}_{y}^{2} + {μ}_{z}^{2}}$ where *qᵢ* and *̅r̅ₗ̅*̅ are the partial charge and position of atom i in the molecule.

The dipole moment is determined by density functional theory (DFT) method.

The geometries of the molecular structures are optimized using the GGA functional BP86 with the Def2-SVP basis set with inclusion of solvent effects via conductor-like polarizable continuum model (CPCM) and as solvent acetonitrile (CPCM(Acetonitrile)) as implemented in the program package ORCA V5.0.3 (Max Planck Institute fuer Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). If more than one conformation is viable, the conformation with the lowest total energy is selected to determine the bond lengths of the molecules. The structures of all the molecules were optimized without symmetry or internal coordinate constrains and were verified as true minima by the absence of negative eigenvalues in the harmonic vibrational frequency analysis.
I. **Geometry optimization** using BP86 functional with def2-SVP basis set and SDD ECPs for elements starting with atomic number 21.
II. **Frequency calculation** using BP86 functional with def2-SVP basis set, and SDD ECPs for elements starting with atomic number 21.
III. **Single Point DFT calculation** using (Gaussian)-B3LYP functional with ZORA-def2-TZVP basis set and auxiliary basis SARC/J.
For the calculations it has been considered for Cu(II) charge 0 and multiplicity 2, for Fe(III) charge 0 and multiplicity 6 (high-spin); temperature is 298.15 K and pressure is 1.0 atm. For all the calculations solvent effects have been included via conductor-like polarizable continuum model (CPCM) with solvent acetonitrile

**Programs:** WEASEL (Version 1.9.2) and ORCA (5.0.3-f.1), for visualization: Chemcraft.

### Calculated HOMO and LUMO of the metal complex of formula (I)

The HOMO and LUMO are calculated with the program package ORCA V5.0.3 (Max Planck Institute fuer Kohlenforschung, Kaiser Wilhelm Platz 1, 45470, Muelheim/Ruhr, Germany) and WEASEL 1.9.2 (FAccTs GmbH, Rolandstrasse 67, 50677 Köln, Germany). The optimized geometries and the HOMO and LUMO energy levels of the molecular structures are determined by applying the hybrid functional (Gaussian)-B3LYP with a ZORA-Def2-TZVP basis set with inclusion of solvent effects via conductor-like polarizable continuum model (CPCM) and as solvent acetonitrile.. If more than one conformation is viable, the conformation with the lowest total energy is selected. The structures of all the molecules were optimized without symmetry or internal constrains and were verified as true minima by the absence of negative eigenvalues in the harmonic vibrational frequency analysis.

### LUMO References: see Table 1

### Experimental data

### Synthesis examples

Compounds of formula (I) may be prepared by known methods or as described below.

### Synthesis of Tris((3-methyl-1-phenyl-4-(2,2,2-trifluoroacetyl)-1H-pyrazol-5-yl)oxy)iron (MC7)

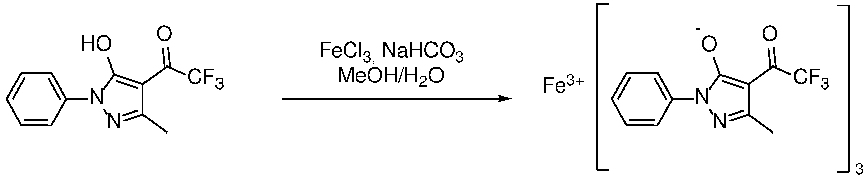

To a mixture of 12.8g (47.4mmol) Ligand and 3.98g (47.4mmol) NaHCO3 in 120ml methanol was added in portions a solution of 2.56g (15.8mmol) of FeC13 in 15ml of water at ice-bath-temperature. The mixture was allowed to stir overnight, then the suspension was filtered and the product washed with little water-methanol. Drying resulted in 12.4g (91%) of the desired product.

### Synthesis of Bis((3-methyl-1-phenyl-4-(2,2,2-trifluoroacetyl)-1H-pyrazol-5-yl)oxy)copper (MC6)

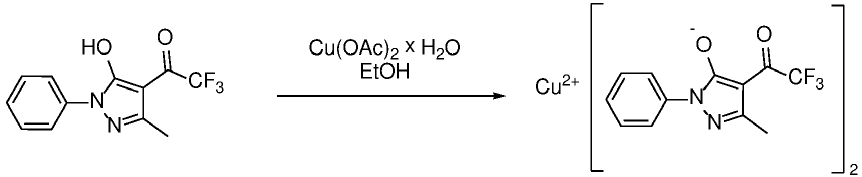

To a solution of 2.5g (9.25mmol) of ligand in 50ml of Ethanol was added 0.92g (4.63mmol) of copper (II) acetate and the mixture was stirred overnight. The resulting green suspension was filtered off and the solid was dried in vacuum to obtain 2.3g (83%) light green-yellowish powder.

### Synthesis of Tetrakis((3-methyl-1-phenyl-4-(2,2,2-trifluoroacetyl)-1H-pyrazol-5-yl)oxy)cerium (MC9)

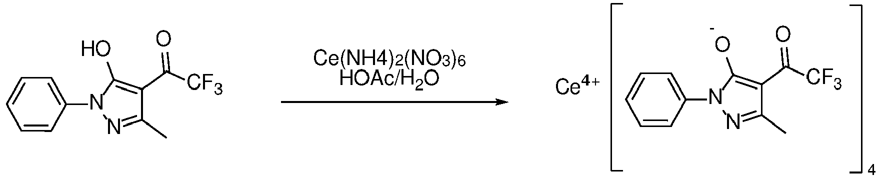

3.76g (13.92mmol) of ligand and 1.07g ammonium acetate (13.92mmol) were dissolved in 50ml acetic acid. 2.86g (5.22mmol) cerium ammonium nitrate were dissolved in 30ml water and added drop wise to the ligand. After 30min the resulting precipitate was filtered off and dried overnight at 100°C in vacuum to obtain 2.63g (62%) black solid.

### Synthesis of 1-(1-(3,5-bis(trifluoromethyl)phenyl)-5-hydroxy-3-methyl-1H-pyrazol-4-yl)-2,2,2-trifluoroethan-1-one

### Step 1. 1-(3,5-bis(trifluoromethyl)phenyl)-3-methyl-1H-pyrazol-5-ol

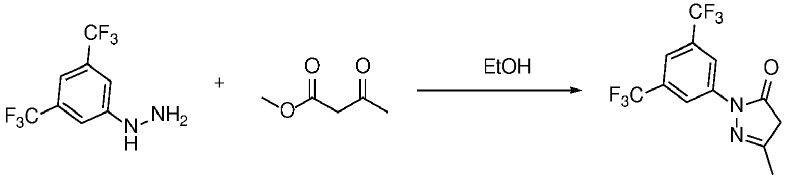

4.0g (16.38mmol) 3,5-bis(trifluoromethyl)phenyl hydrazine were dissolved in 60ml ethanol and a solution of 1.9g (16.38mmol) methyl acetoacetate was added dropwise. The reactions for heated to reflux for 4h. After removing the solvent, the residue was distilled from bulb to bulb in vacuum. The residue was further purified by flash chromatography using ethyl acetate/hexane and sublimation to obtain 2.33g (46%) slightly yellow, crystalline solid.

### Step 2. 1-(1-(3,5-bis(trifluoromethyl)phenyl)-5-hydroxy-3-methyl-1H-pyrazol-4-yl)-2,2,2-trifluoroethan-1-one

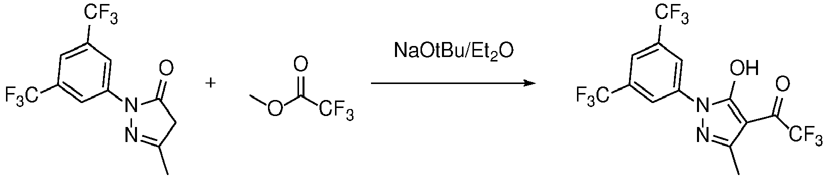

1.38g (14.38mmol) sodium tert-butylate and 1.1g (8.63mmol) trifluoroaceticacid methyl ester were dissolved in 10ml dry diethyl ether and cooled with an ice bath. A solution of 2.23g (7.19mmol) 1-(3,5-bis(trifluoromethyl)phenyl)-3-methyl-1H-pyrazol-5-ol in 15ml diethyl ether was added dropwise. The mixture was stirred at room temperature overnight. After drying with magnesium sulfate the solvent was removed and the crude product sublimed to obtain 2.1g (72%) slightly reddish solid.

### Synthesis of Bis((1-(3,5-bis(trifluoromethyl)phenyl)-3-methyl-4-(2,2,2-trifluoroacetyl)-1H-pyrazol-5-yl)oxy)copper (MC56)

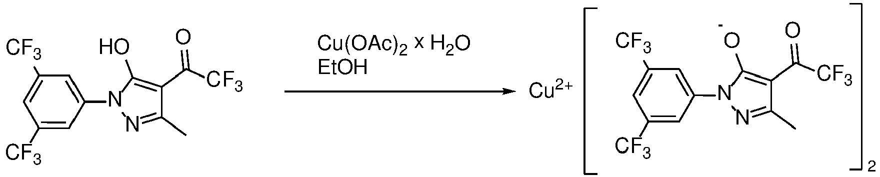

2.0g (4.92mmol) ligand was dissolved in 20ml ethanol and 0.49g (2.46mmol) copper (II) acetate were added. The mixture was stirred overnight at room temperature, cooled with an ice bath and the precipitate was filtered off, washed with ethanol and dried at 70°C in vacuum to obtain 1.95g (91%) bright green powder.

Further compounds according to invention may be prepared by the methods described above or by methods known in the art.

Further compounds according to invention may be prepared by the methods described above or by methods known in the art.

### General procedure for fabrication of OLEDs

### General procedure for fabrication of organic electronic devices comprising a semiconductor layer comprising a metal complex and a matrix compound wherein the semiconductor layer is deposited in vacuum

For inventive examples 1-1 to 1-21 and comparative example 1-1 in Table 3 a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment to prepare the anode layer. The plasma treatment was performed in an atmosphere comprising 97.6 vol.-% nitrogen and 2.4 vol.-% oxygen.

Then, the metal complex and the matrix compound were co-deposited in vacuum on the anode layer, to form a hole injection layer (HIL) having a thickness of 10 nm. The composition of the hole injection layer can be seen in Table 3. The formulae of the metal complexes can be seen in Table 1.

Then, the matrix compound was vacuum deposited on the HIL, to form a HTL having a thickness of 123 nm. The compound of formula (II) in the HTL is selected the same as the matrix compound in the HIL. The matrix compound can be seen in Table 3.

Then N-(4-(dibenzo[b,d]furan-4-yl)phenyl)-N-(4-(9-phenyl-9H-fluoren-9-yl)phenyl)-[1,1'-biphenyl]-4-amine (CAS 1824678-59-2) was vacuum deposited on the HTL, to form an electron blocking layer (EBL) having a thickness of 5 nm.

Then 97 vol.-% H09 as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Ag:Mg (90:10 vol.-%) was evaporated at a rate of 0.01 to 1 Å/s at 10⁻⁷ mbar to form a cathode layer with a thickness of 13 nm on the electron transporting layer.

Then, K1 was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

In bottom emission devices, the emission is predominately Lambertian and quantified in percent external quantum efficiency (EQE). The light is emitted through the anode layer. To determine the efficiency EQE in % the light output of the device is measured using a calibrated photodiode at 10 mA/cm2.

In top emission devices, the emission is forward directed through the cathode layer, non-Lambertian and also highly dependent on the mirco-cavity. Therefore, the efficiency EQE will be higher compared to bottom emission devices. To determine the efficiency EQE in % the light output of the device is measured using a calibrated photodiode at 10 mA/cm².

Lifetime LT of the device is measured at ambient conditions (20°C) and 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

To determine the voltage stability over time U(100h)-(1h), a current density of at 30 mA/cm² was applied to the device. The operating voltage was measured after 1 hour and after 100 hours, followed by calculation of the voltage stability for the time period of 1 hour to 100 hours. A low value for U(100h)-(1h) denotes a low increase in operating voltage over time and thereby improved voltage stability.

Conductivity is determined by transmission line method. Voltage between -5 and +5V in steps of 0.5V is applied on a transmission line covered with a homogeneous layer of studied material having a uniform thickness. The current is measured using a Keithley SM2635 source measure unit. Conductivity is calculated using known geometry of the transmission line and thickness of the deposited layer.

### General method for preparation of the ink formulation

To prepare the ink formulation, the compounds were weighed into vials. Then, the solvent was added. The mixture was stirred for 10 min. The inks were transferred to inert atmosphere. An aliquot of benzonitrile solutions was added to the anisole solution to obtain a solution with a ratio of 5:1 of anisole to benzonitrile solution. The resulting solution was stirred again for at least 10 min at room temperature. The resulting ink formulation had a solid content of 4 wt.-%.

### Ink formulation for inventive example 2-1

The ink formulation for example 2-3 has the following composition: 4 wt.-% K1 in anisole : benzonitrile (5:1). To prepare the ink, solution of 166 mg K1 in 3.3 ml anisole were prepared as described above. 0.7 ml benzonitrile was added to the anisole solution and stirred as described above.

### Ink formulation for inventive example 2-1

The ink formulation for example 2-1 has the following composition: 4 wt.-% MC-9 : K1 in anisole : benzonitrile (5:1). To prepare the ink, solutions of 12,6 mg (1 mol. %, 1.78 wt. %) MC9 in 3 ml benzonitrile and 163 mg K1 in 3.3 ml anisole were prepared as described above. 0.7 ml benzonitrile solution was added to the anisole solution and stirred as described above.

### Ink formulation for inventive example 2-3

The ink formulation for example 2-2 has the following composition: 4 wt.-% MC-2 : K1 in anisole : benzonitrile (5:1). To prepare the ink, solutions of 24 mg (1 mol. %, 5.25 wt. %) MC9 in 2 ml benzonitrile and 158 mg K1 in 3.3 ml anisole were prepared as described above. 0.7 ml benzonitrile solution was added to the anisole solution and stirred as described above.

### General procedure for fabrication of electronic devices comprising a semiconductor layer comprising a metal complex and a matrix compound wherein the semiconductor layer is deposited from solution

For conductivity devices, see Examples 2-1, 2-2 and 2-3 in Table 4, a 15Ω /cm² glass substrate with 90 nm ITO (available from Corning Co.) with the dimensions 150 mm x 150 mm x 0.7 mm was ultrasonically washed with isopropyl alcohol for 5 minutes and then with pure water for 5 minutes and dried at elevated temperature.

To form the organic layer, the substrate is placed on a spin-coater with ITO side facing upwards and fixed with vacuum. 4 ml of ink formulation is applied with a syringe with filter (PTFE - 0.2µm) on the substrate. Spin-coating parameter are 650 rpm (3sec ramp-up from zero to maximum speed) for 30sec. The resulting film is dried at 60°C for 1 minute on a hotplate. Next step is the cleaning of the substrate around the active area (to ensure a good encapsulation before measurement). An additional bake-out at 100°C for 10 minutes on a hotplate is done. The composition of the organic layer can be seen in Table 4. The formulae of the metal complexes can be seen in Table 1.

Then, the substrate is transferred to another inert glovebox for encapsulation.

Then, Biphenyl-4-yl(9,9-diphenyl-9H-fluoren-2-yl)-[4-(9-phenyl-9H-carbazol-3-yl) phenyl]-amine was vacuum deposited on the HIL, to form a first HTL having a thickness of 89 nm.

### Technical Effect of the invention

In Table 1 are shown the calculated LUMO in electron volt of metal complexes of formula (I). Where more than one spin state is viable, the spin state is stated in brackets. As can be seen in Table 1, the LUMO energy are within the range suitable for organic electronic devices.

Also in Table 1 are shown calculated LUMO in electron volt of comparative metal complexes CC1 to CC5. The same method was used as for metal complexes of formula (I). As can be seen in Table 1, the LUMO energies of known acetyl pyrazolonate compounds CC1-CC3 are closer to vacuum level than LUMO energies of the inventive acetylpyrazolonate compounds.

Without being bound by theory, a LUMO energies further away from vacuum level is beneficial for improved performance of organic electronic devices.

In Table 2 are shown data for top emission organic electronic devices fabricated by co-deposition from vacuum of metal complex and matrix compound.

In comparative example 1-1, a metal complex known in the art is tested at 10 wt.- %.

As can be seen in Table 2, in comparative example 1-1 the operating voltage is 3.75 V, the external quantum efficiency EQE is 14.5 % and the voltage stability over time is 1.988 V.

In inventive example 1-1, the semiconductor layer comprises a compound of formula (I) MC6. MC6 is a metal complex of formula (I). As can be seen in Table 2, the operating voltage is improved to 3.71 V, cd/A efficiency is 6.5 cd/A, the EQE is 14.4 % and operating voltage stability over time is improved to 0.184 V.

In inventive examples 1-2, the semiconductor layer comprises 15 vol.-% MC6. As can be seen in Table 2, the operating voltage, cd/A efficiency, EQE, and voltage stability over time are further improved.

In inventive examples 1-3 to 1-5, the semiconductor layer comprises metal complex of formula (I) MC7. As can be seen in Table 2, the operating voltage, cd/A efficiency, EQE, lifetime and/or operating voltage stability over time are improved over comparative example 1-1.

In inventive examples 1-6 to 1-17, the semiconductor layer comprises metal complexes of formula (I) and various matrix compounds, wherein the HOMO of the matrix compound is further away from vacuum level compared to comparative example 1 and inventive examples 1-1 to 1-5. As can be seen in Table 2, the operating voltage, cd/A efficiency, EQE, lifetime and/or operating voltage stability over time are improved over comparative example 1-1.

Improvements in performance of inventive organic electronic devices can be observed also in comparison with another prior art metal complex CC5 used as a p-dopant in comparative examples 1-2 to 1-8.

In Table 3 are shown data for organic electronic conductivity devices fabricated by co-deposition from solution of compound of formula (I) and a matrix compound.

As can be seen in Table 3, a significant conductivity increase of the materials doped with inventive compound MC9 in comparison with undoped matrix compound is obtained.

A low operating voltage, high efficiency, high lifetime and/or improved operating voltage stability over time are important for the performance and long-term stability of organic electronic devices.

**Table 2: Performance of an organic electroluminescent device comprising a semiconductor laver comprising a metal complex of formula 1**

| | Metal compl ex | Percentage metal complex in semiconduc tor layer [wt.-%] | Matrix compou nd | HOMO level of matrix compou nd [eV] | Percentage matrix compound in semiconductor layer [wt.-%] | Semicond uctor layer thickness [nm] | Voltage at 10mA/c m² [V] | Ceff at 10mA/c m² [cd/A] | EQE at 10mA/c m² [%] | LT97 at 30mA/c m² [h] | Vrise (1-100h) at 30mA/c m² [V] |
|---|---|---|---|---|---|---|---|---|---|---|---|
| Comparati ve example 1-1 | CC4 | 10 | K1 | -5.04 | 90 | 10 | 3.75 | 6.5 | 14.5 | 136 | 1.988 |
| Comparati ve example 1-2 | CC5 | 10 | K1 | -5.04 | 90 | 10 | 3.76 | 6.5 | 14.5 | 117 | 0.634 |
| Comparati ve example 1-3 | CC5 | 10 | K16 | -5.14 | 90 | 10 | 3.93 | 6.8 | 14.7 | 31 | 1.204 |
| Comparati ve example 1-4 | CC5 | 20 | K16 | -5.14 | 80 | 10 | 3.78 | 7.0 | 15.2 | 43 | 1.487 |
| Comparati ve example 1-5 | CC5 | 30 | K16 | -5.14 | 70 | 10 | 3.78 | 6.8 | 15.0 | 24 | 2.175 |
| Comparati ve example 1-6 | CC5 | 10 | K2 | -5.19 | 90 | 10 | 4.43 | 6.2 | 13.8 | 11 | 2.152 |
| Comparati ve example 1-7 | CC5 | 20 | K2 | -5.19 | 80 | 10 | 4.14 | 5.7 | 12.9 | 8 | 2.815 |
| Comparati ve example 1-8 | CC5 | 30 | K2 | -5.19 | 70 | 10 | 3.91 | 5.9 | 13.1 | 8 | 3.300 |
| Inventive example 1-1 | MC6 | 10 | K1 | -5.04 | 90 | 10 | 3.71 | 6.5 | 14.4 | 103 | 0.184 |
| Inventive example 1-2 | MC6 | 15 | K1 | -5.04 | 85 | 10 | 3.70 | 6.6 | 14.6 | 89 | 0.075 |
| Inventive example 1-3 | MC7 | 5 | K1 | -5.04 | 95 | 10 | 3.73 | 6.5 | 14.3 | 110 | 0.406 |
| Inventive example 1-4 | MC7 | 10 | K1 | -5.04 | 90 | 10 | 3.69 | 6.5 | 14.5 | 90 | 0.072 |
| Inventive example 1-5 | MC7 | 20 | K1 | -5.04 | 80 | 10 | 3.69 | 6.5 | 14.4 | 91 | 0.051 |
| Inventive example 1-6 | MC6 | 10 | K16 | -5.14 | 90 | 10 | 3.68 | 7.1 | 16.1 | 77 | 0.186 |
| Inventive example 1-7 | MC6 | 15 | K16 | -5.14 | 85 | 10 | 3.68 | 7.2 | 16.0 | 76 | 0.093 |
| Inventive example 1-8 | MC6 | 20 | K16 | -5.14 | 20 | 10 | 3.67 | 7.2 | 16.0 | 75 | 0.080 |
| Inventive example 1-9 | MC7 | 10 | K16 | -5.14 | 90 | 10 | 3.69 | 7.3 | 15.6 | 78 | 0.265 |
| Inventive example 1-10 | MC7 | 20 | K16 | -5.14 | 80 | 10 | 3.67 | 7.2 | 15.6 | 75 | 0.101 |
| Inventive example 1-11 | MC7 | 30 | K16 | -5.14 | 70 | 10 | 3.67 | 7.3 | 15.6 | 71 | 0.118 |
| Inventive example 1-12 | MC56 | 10 | K16 | -5.14 | 90 | 10 | 3.69 | 7.5 | 16.7 | 75 | 0.291 |
| Inventive example 1-13 | MC6 | 10 | K2 | -5.19 | 90 | 10 | 3.66 | 7.1 | 15.3 | 187 | 0.704 |
| Inventive example 1-14 | MC6 | 15 | K2 | -5.19 | 85 | 10 | 3.62 | 7.1 | 15.5 | 115 | 0.077 |
| Inventive example 1-15 | MC7 | 5 | K2 | -5.19 | 95 | 10 | 3.64 | 7.0 | 15.4 | 121 | 0.089 |
| Inventive example 1-16 | MC7 | 10 | K2 | -5.19 | 90 | 10 | 3.61 | 7.0 | 15.4 | 118 | 0.035 |
| Inventive example 1-17 | MC7 | 15 | K2 | -5.19 | 85 | 10 | 3.62 | 7.0 | 15.4 | 121 | 0.029 |
| Inventive example 1-18 | MC6 | 100 | n.a. ¹⁾ | n.a. | n.a. | 3 | 3.71 | 6.3 | 14.5 | 87 | 0.048 |
| Inventive example 1-19 | MC6 | 100 | n.a. | n.a. | n.a. | 5 | 3.73 | 6.2 | 14.7 | 89 | 0.012 |
| Inventive example 1-20 | MC7 | 100 | n.a. | n.a. | n.a. | 2 | 3.71 | 6.6 | 14.6 | 79 | 0.047 |
| Inventive example 1-21 | MC7 | 100 | n.a. | n.a. | n.a. | 3 | 3.71 | 6.4 | 14.5 | 81 | 0.062 |

| | | | | | | | | | | | |
|---|---|---|---|---|---|---|---|---|---|---|---|
| ¹⁾ n.a. = not applicable | | | | | | | | | | | |

**Table 3: Conductivity of p-doped materials prepared via deposition from solution**

| | Metal complex | Percentage metal complex in semiconductor layer [wt.-%] | Matrix compound | Percentage matrix compound in semiconductor layer [wt.-%] | **Current(5 V, 40 µm) [µA]** | **Conductivity [S/cm]** |
|---|---|---|---|---|---|---|
| Comparative example 2-1 | none | 0 | K1 | 100% | 9e-6 | 5.8e-11 |
| Inventive example 2-2 | MC9 | 1.78 | K1 | 98.22 | 0.39 | 2.58e-6 |
| Inventive example 2-2 | MC9 | 5.25 | K1 | 94.75 | 1.41 | 9.1e-6 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. A metal complex of formula (I)
M^{n_{⊕}}(L^{Θ})n(AL)m (I),
wherein
M is a metal ion;
n is the valency of M and selected from 1 to 4;
L has formula (II) 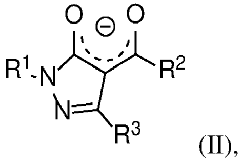
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
R³ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein at least one of R¹, R² and R³ or the group of R¹, R² and R³ comprises at least three atoms selected from the group consisting of halogen, Cl, F or N;
wherein
AL is an ancillary ligand which coordinates to the metal M;
m is an integer selected from 0 to 2.

2. The metal complex of formula (I) according to claim 1, wherein at least one of R¹, R² and R³ or the group of R¹, R² and R³ comprises at least three atoms selected from the group consisting of halogen, Cl, F or N.

3. The metal complex of formula (I) according to claim 1 or 2, wherein
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl that comprises at least one substituted or unsubstituted 6-membered heteroaryl;
R³ is selected from partially or perfluorinated C₁ to C₄ alkyl or CN.

4. The metal complex of formula (I) according to claims 1 to 3, wherein the group consisting of R¹, R² and R³ comprises:
- at least two moieties selected from CF₃ or CN; or
- at least one moiety selected from CF₃ or CN and one substituted or unsubstituted 6-membered heteroaryl; or
- R³ is CN.

5. The metal complex of formula (I) according to claims 1 to 4, wherein at least one of the group consisting of R¹, R² and R³ is selected from CN, CH₃, CF₃, C₂F₅, C₃F₇ or C₄F₉, preferably CN, CH₃, CF₃, C₂F₅ or C₃F₇; more preferred CN, CH₃ or CF₃.

6. The metal complex of formula (I) according to claims 1 to 5, wherein the metal complex of formula (I) comprises at least one CF₃ group, preferably at least two CF₃ groups, further preferred at least three CF₃ groups, in addition preferred at least four CF₃ groups, or at least two CF₃ groups and at least one C₂F₅, or at least two CF₃ groups and at least one group selected from CN, F or CH₃.

7. The metal complex of formula (I) according to claims 1 to 6, wherein at least one of the group consisting of R¹, R² and R³ is selected from a substituted or unsubstituted C₆ to C₁₉ aryl or a substituted C₂ to C₂₀ heteroaryl group comprising at least 6 ring-forming atoms, and is selected from the following Formulae D1 to D71: 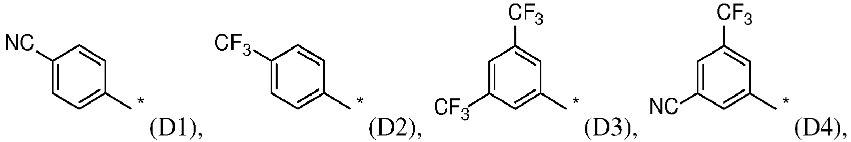 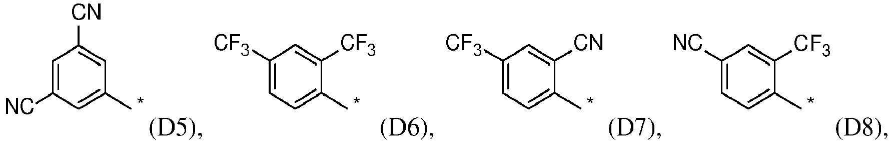 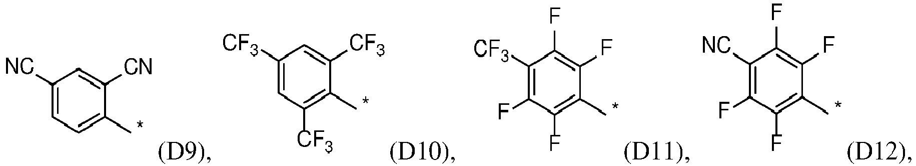 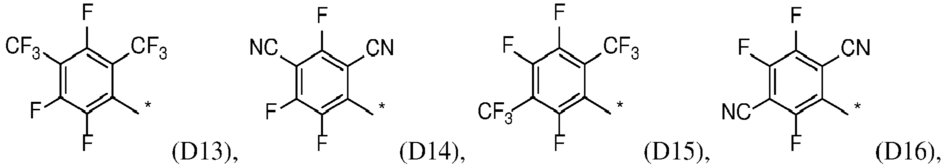 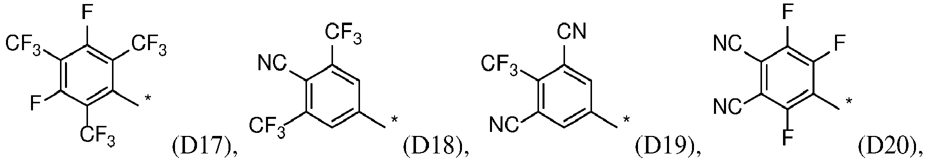 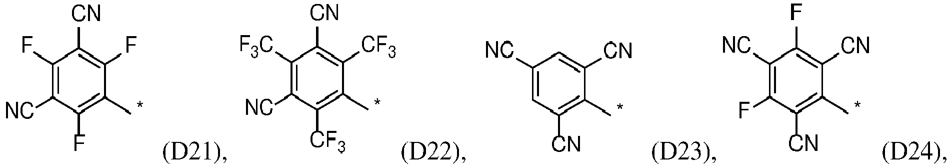 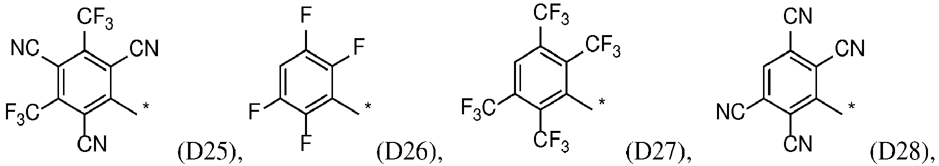 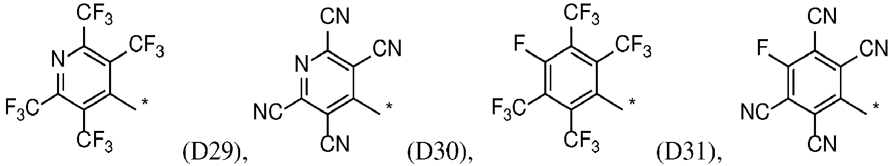 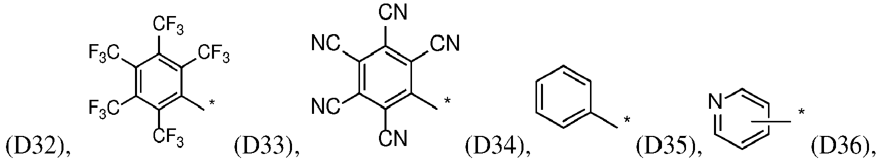 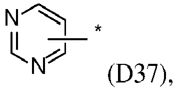 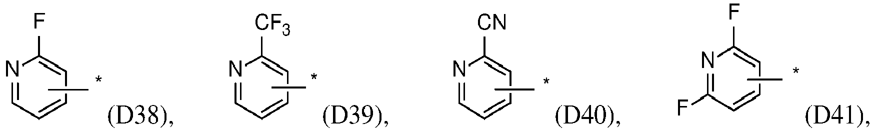 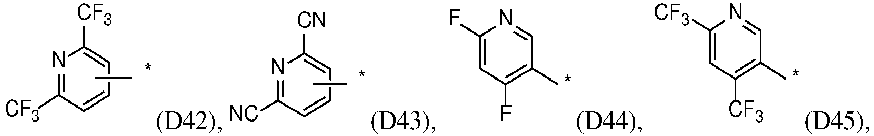 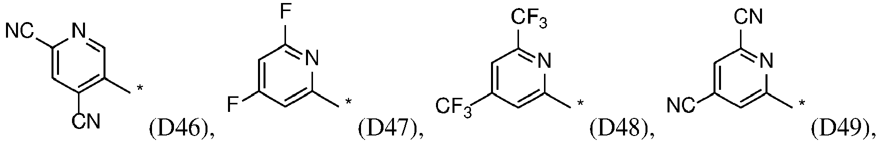 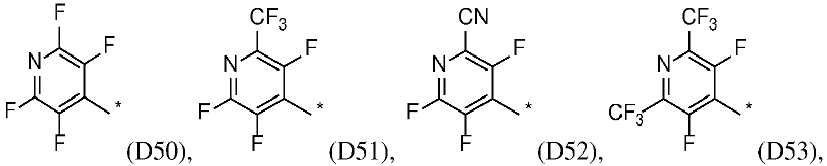 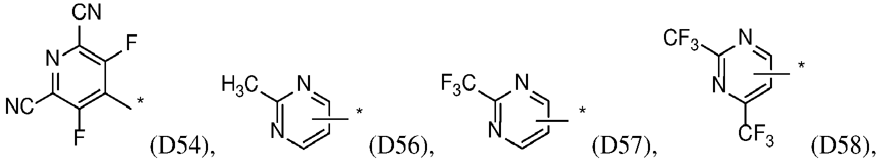 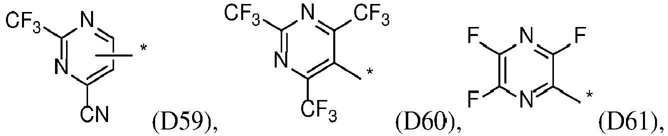 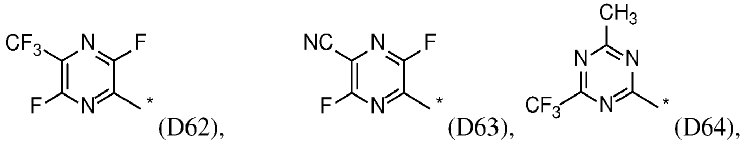 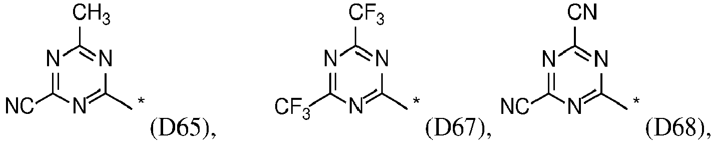 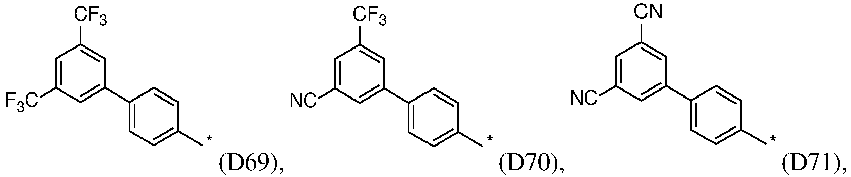 wherein the "^{∗}" denotes the binding position.

8. The metal complex of formula (I) according to claims 1 to 7, wherein M is a metal ion selected from an alkali, alkaline earth, transition, rare earth metal or group III to V metal, preferably M is a metal ion selected from transition or group III to V metal; preferably M is a metal ion selected from Li(I), K(I), Rb(I), Cs(I), Ag(I), Cu(II), Zn(II), Pd(II), Ir(III), Al(III), Sc(III), Mn(III), Ru(III), In(III), Y(III), Eu(III), Fe(III), V(IV), Zr(IV), Hf(IV) and Ce(IV); more preferred M is a metal ion selected from Li(I), K(I), Ag(I), Cu(II), Zn(II), Al(III), Sc(III), Mn(III), Fe(III), Zr(IV), and Ce(IV); also preferred M is Cu(II) or Fe(III), and further more preferred M is Cu(II); wherein the number in brackets denotes the oxidation state.

9. The metal complex of formula (I) according to claims 1 to 8, wherein the Ligand L is selected from the group comprising the following Formulae E1 to E211:

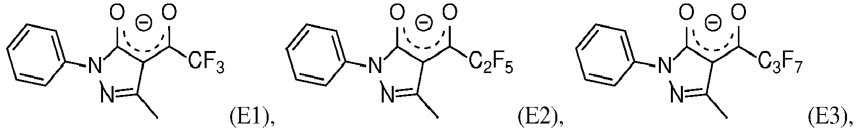

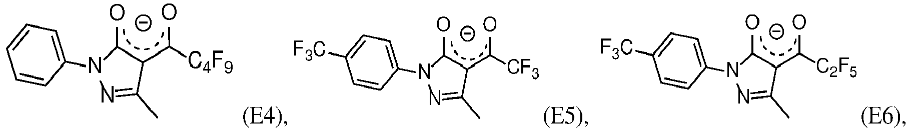

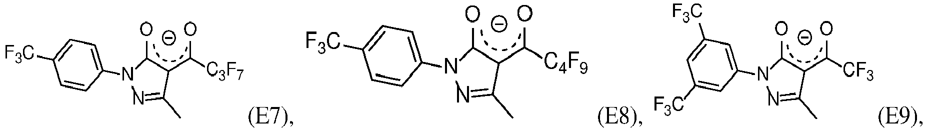

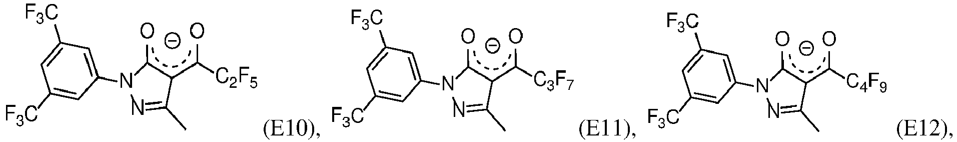

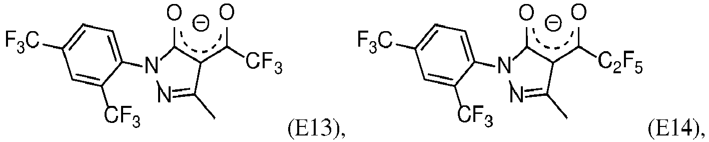

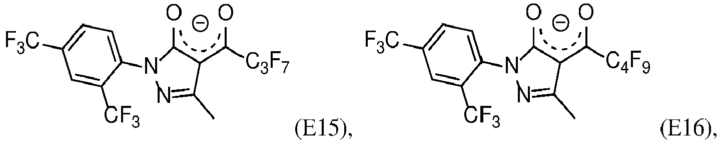

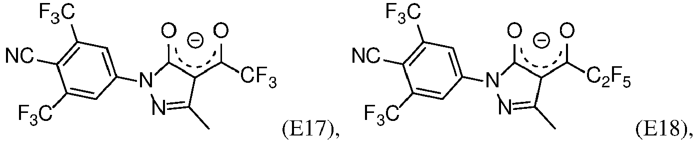

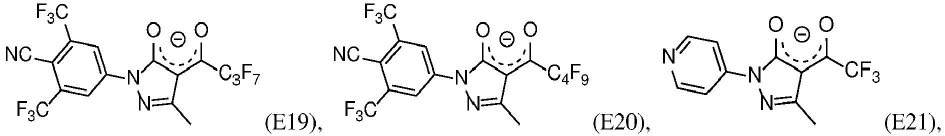

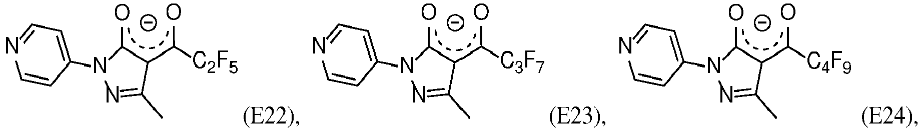

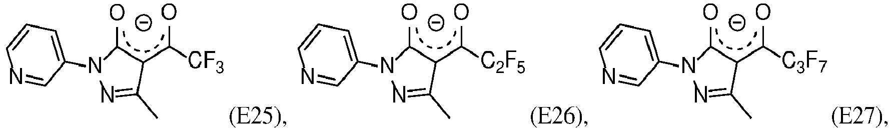

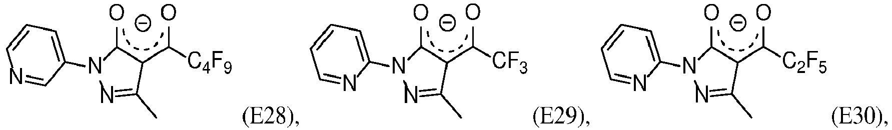

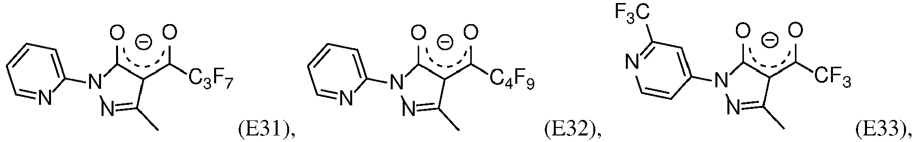

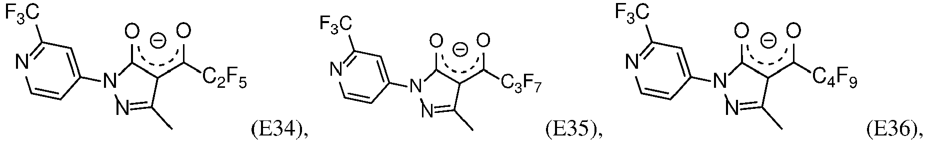

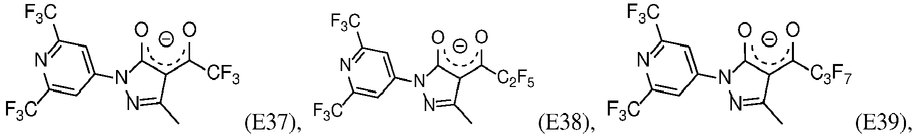

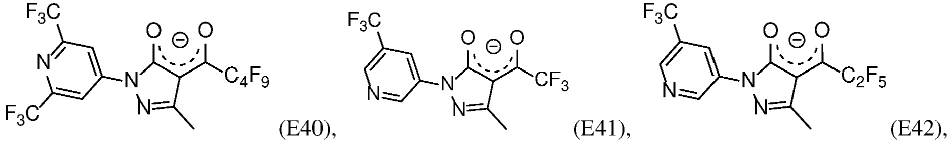

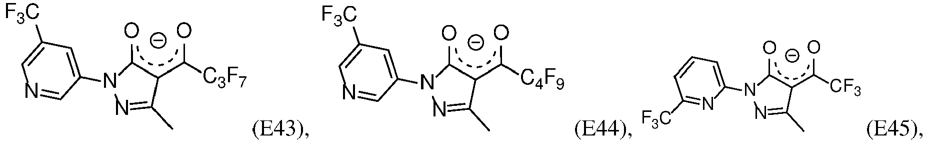

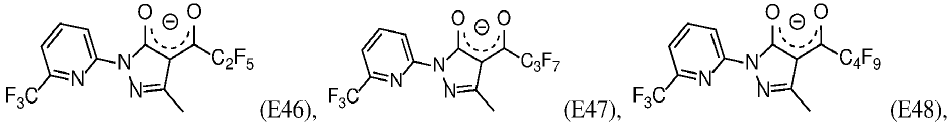

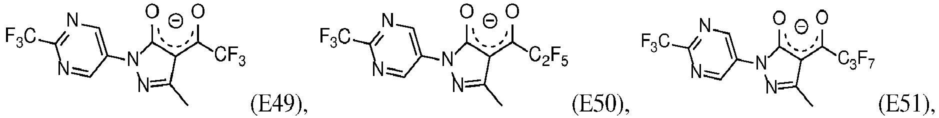

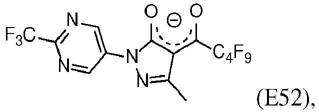

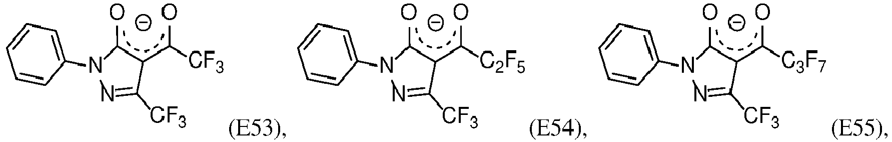

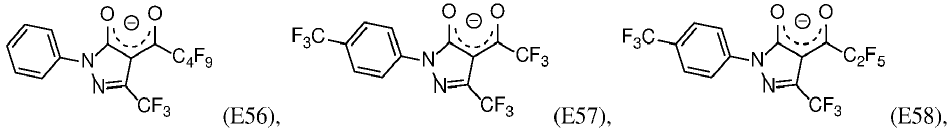

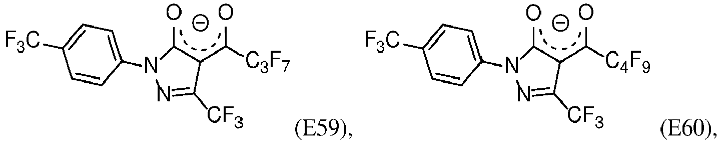

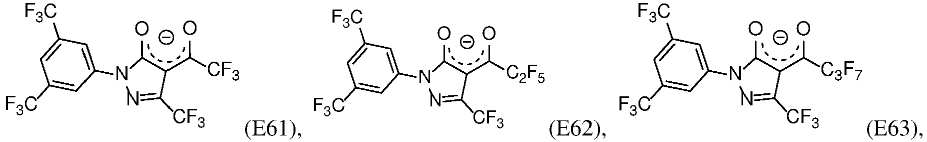

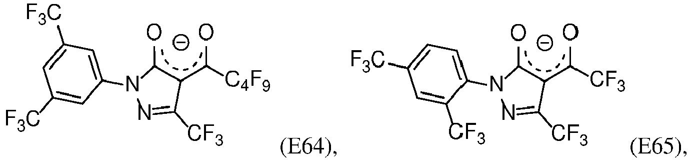

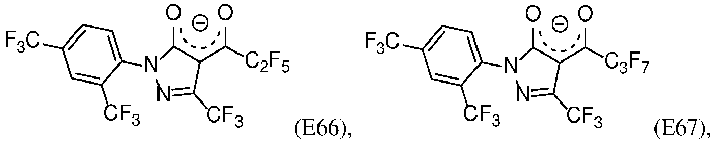

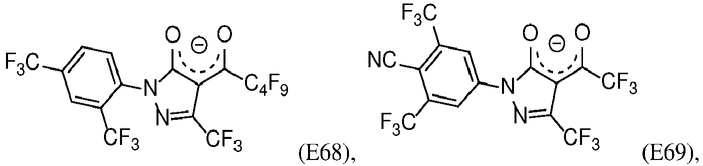

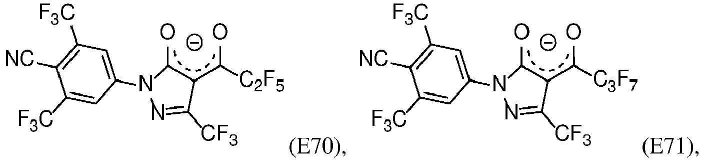

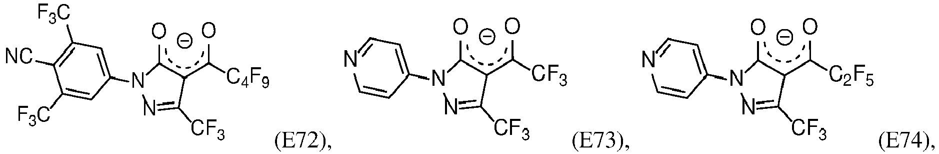

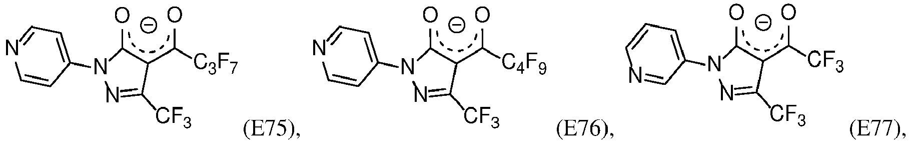

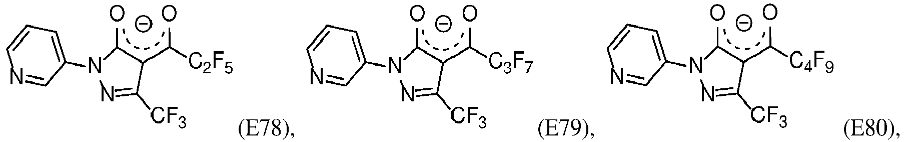

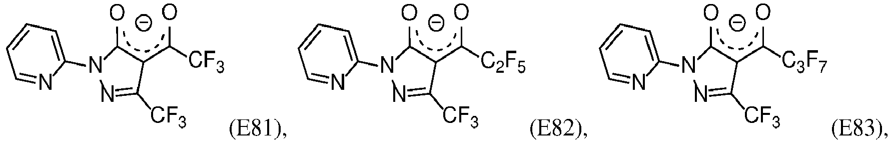

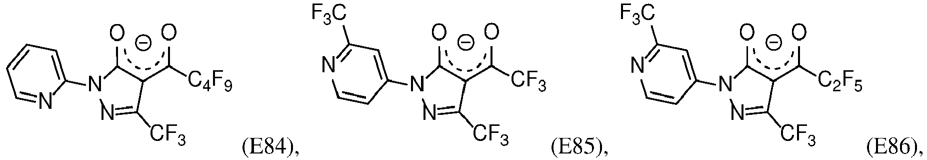

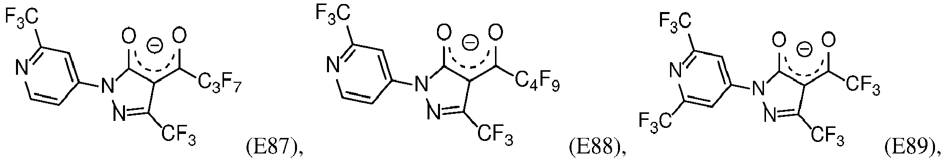

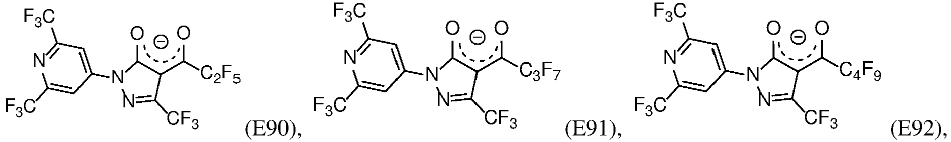

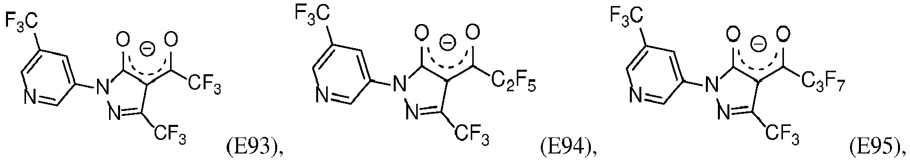

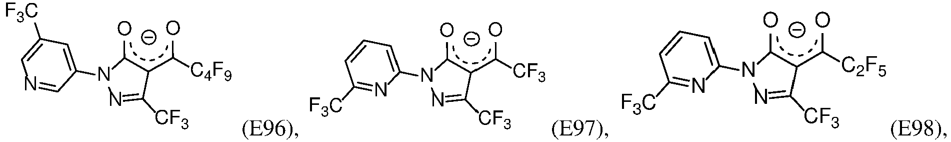

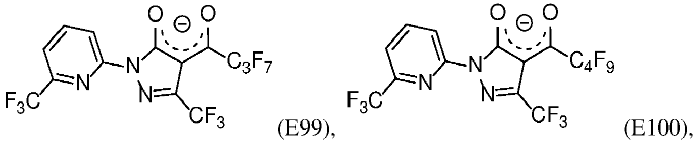

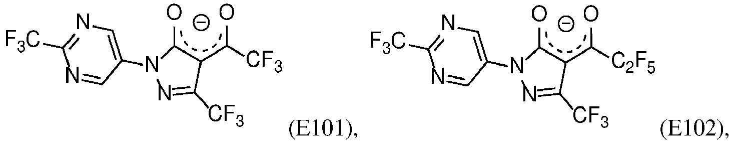

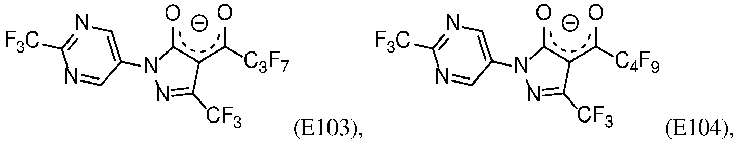

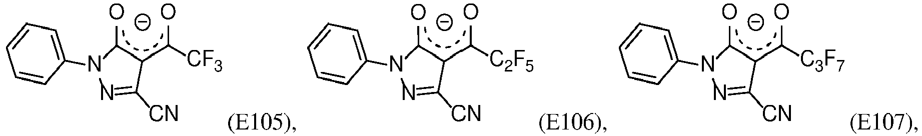

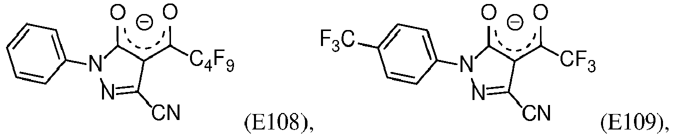

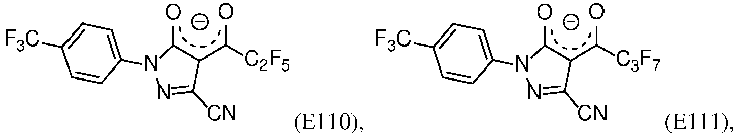

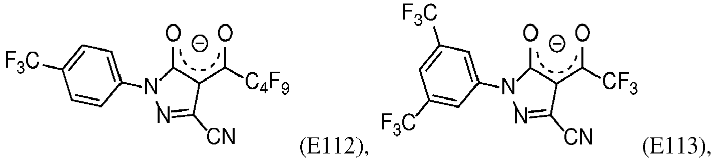

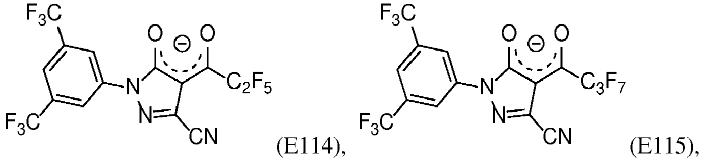

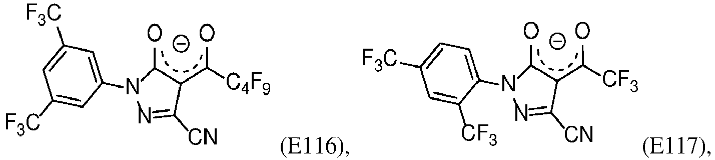

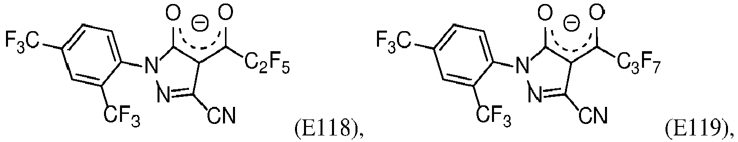

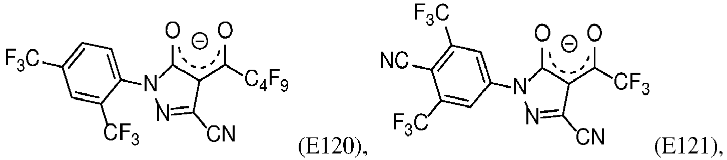

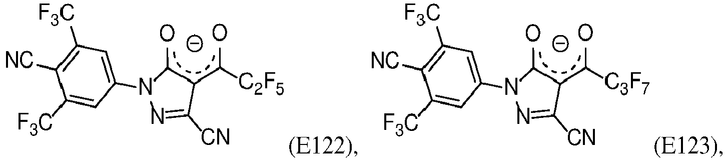

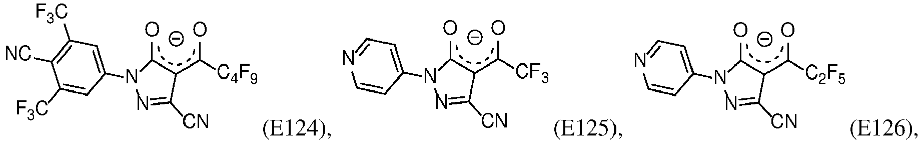

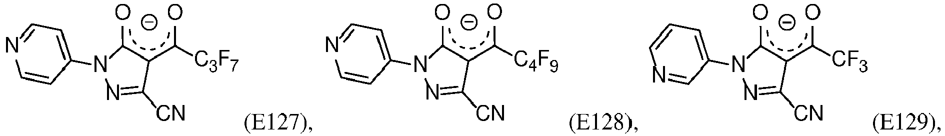

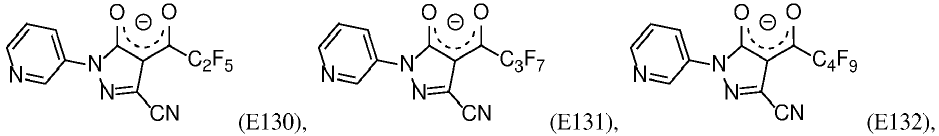

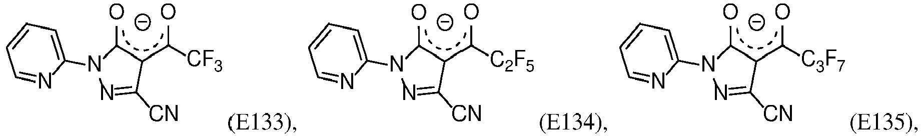

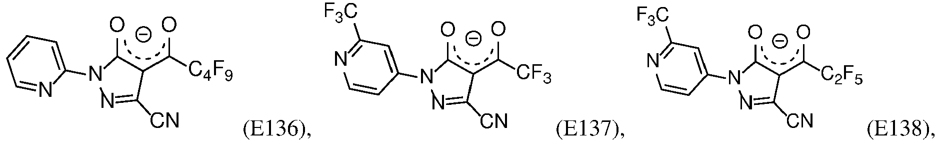

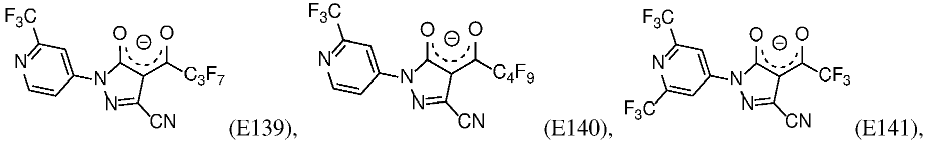

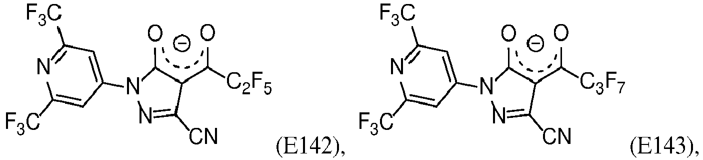

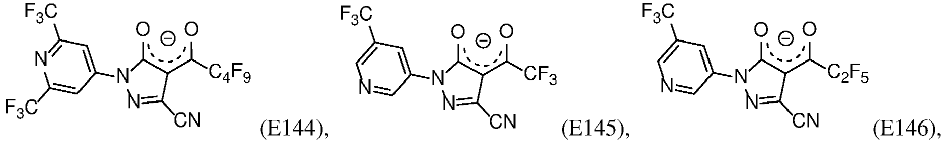

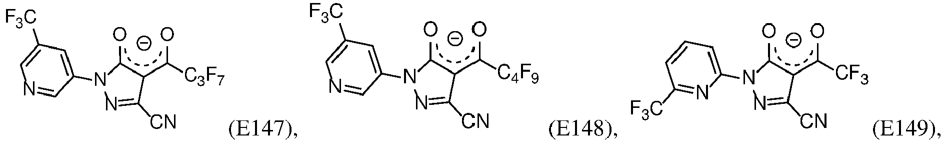

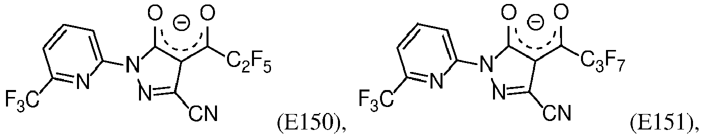

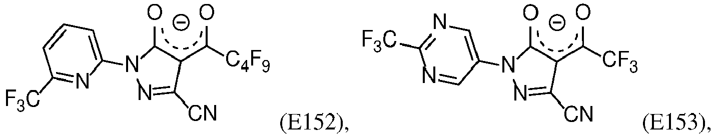

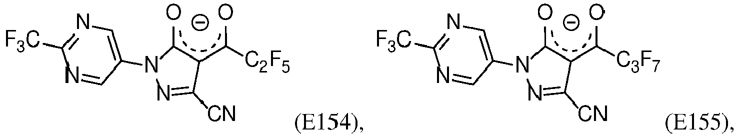

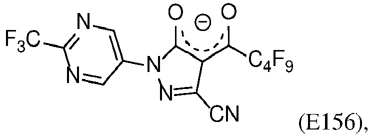

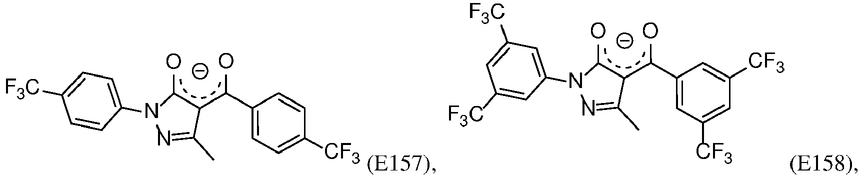

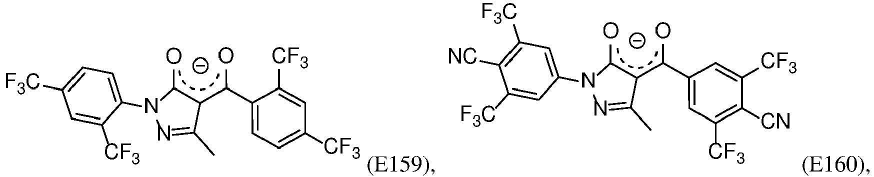

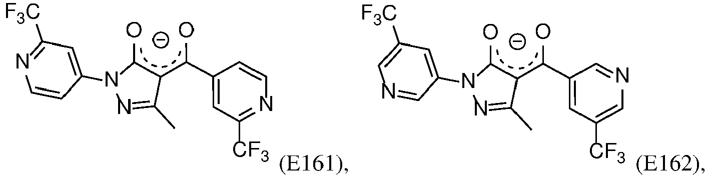

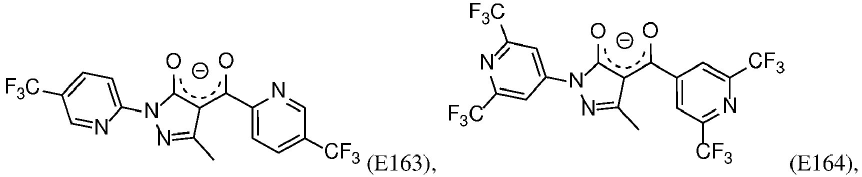

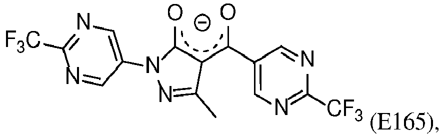

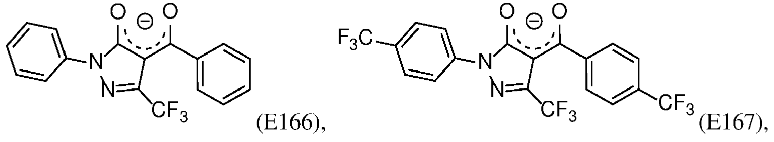

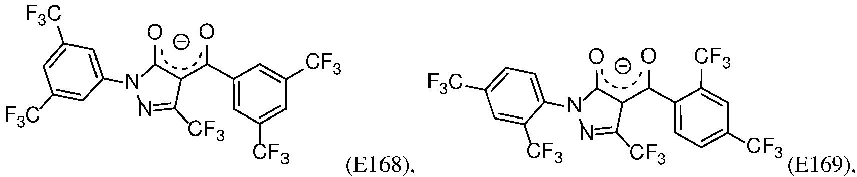

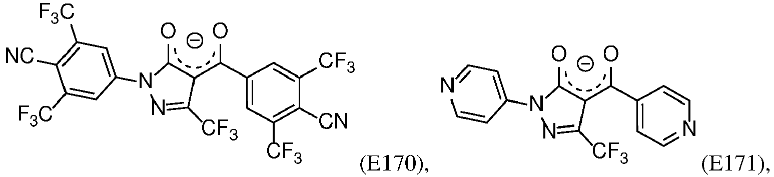

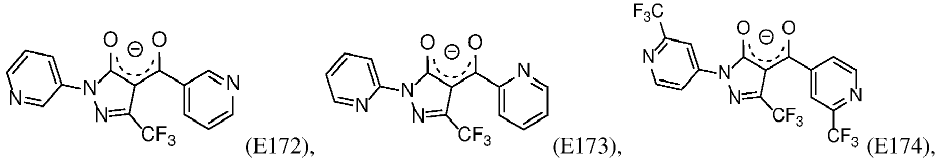

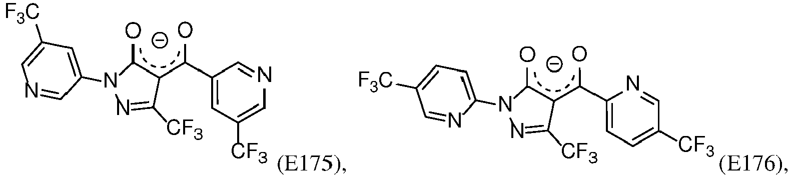

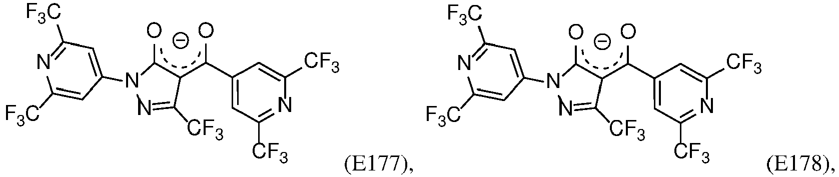

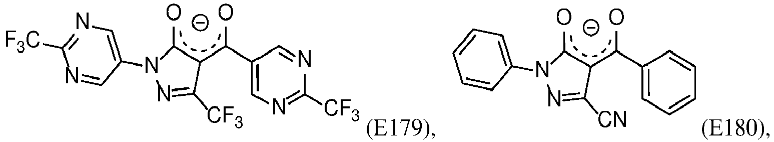

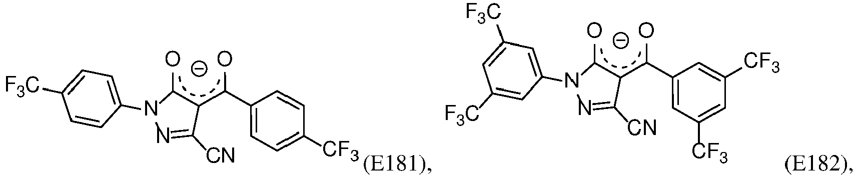

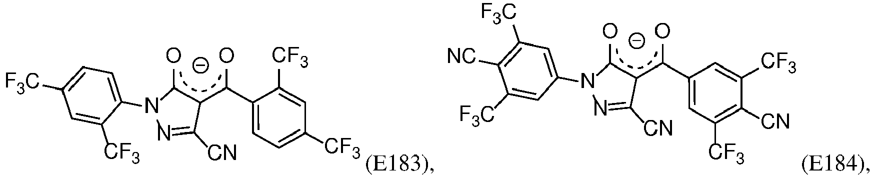

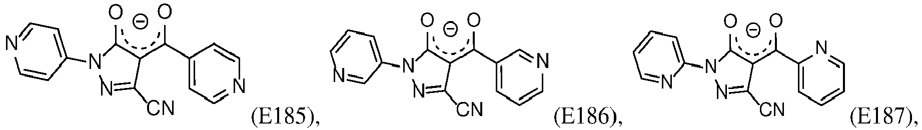

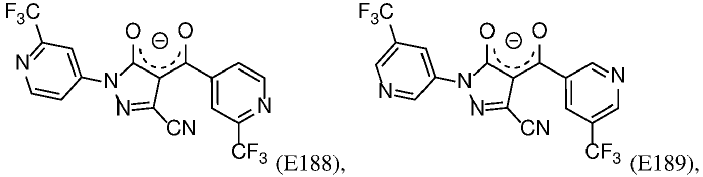

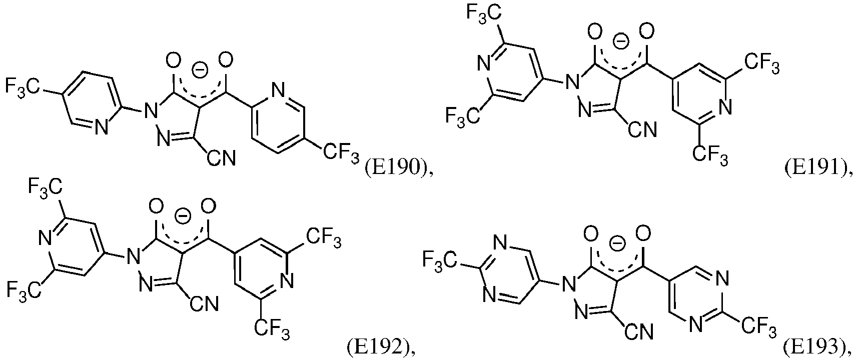

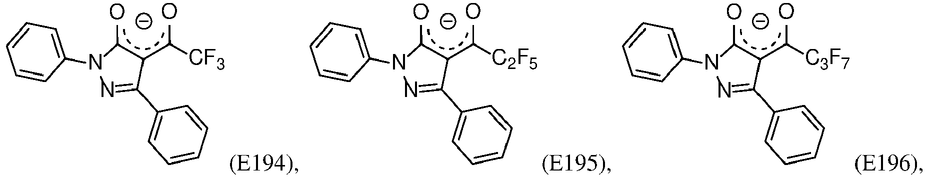

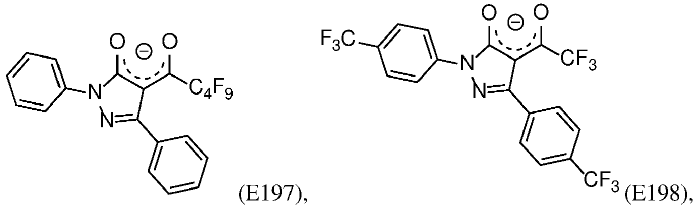

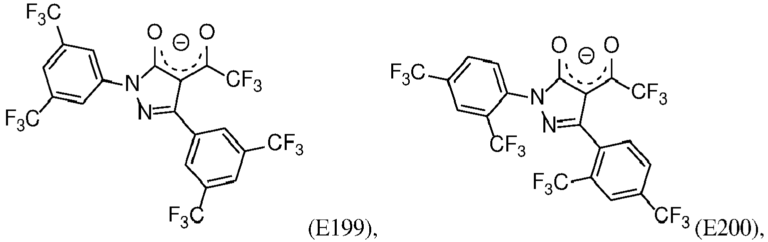

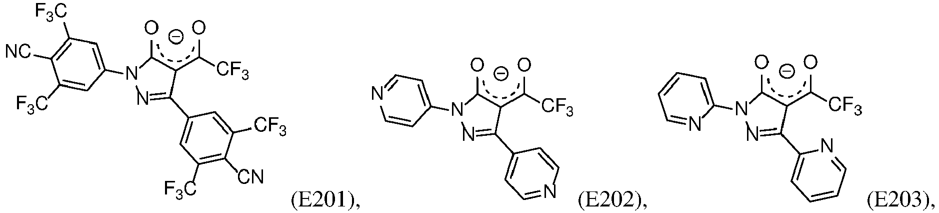

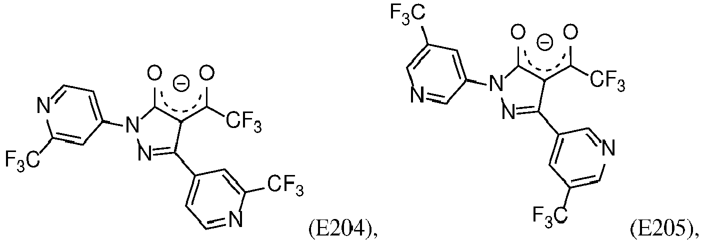

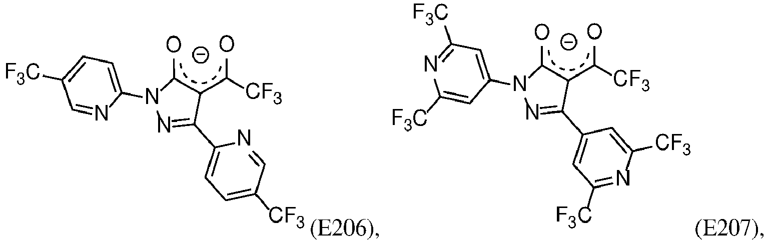

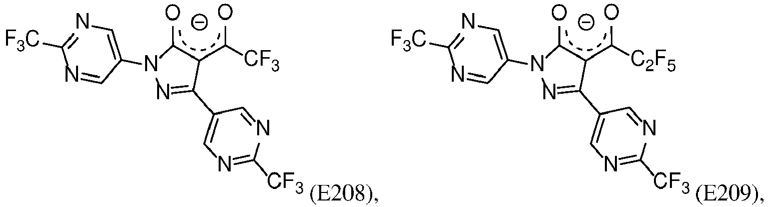

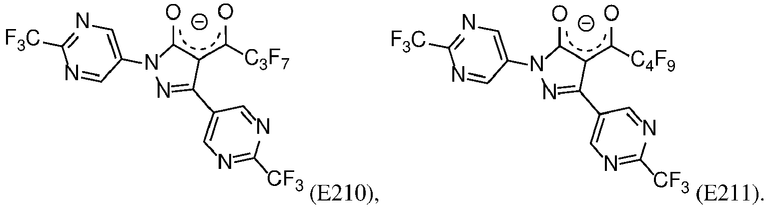

10. The metal complex of formula (I) according to claims 1 to 9, wherein AL is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (III); 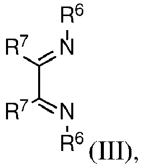 wherein
R⁶ and R⁷ are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

11. The metal complex of formula (I) according to any of claims 1 to 10, wherein
- n = 2 or 3; and/or
- m is an integer selected from 0 or 1, preferably 0.

12. The metal complex of formula (I) of Formula (I) according to any of claims 1 to 11, wherein at least one R¹ or R³, or R¹ and R³ are selected from a substituted C₂ to C₂₀ heteroaryl group comprising at least 6 ring-forming atoms, wherein the C₂ to C₂₀ heteroaryl group comprising at least 6 ring-forming atoms is selected from pyridyl, pyrimidinyl, pyrazinyl, triazinyl.

13. The metal complex of formula (I) according to claims 1 to 12:
M^{n_{⊕}}(L^{Θ})n(AL)m (I),
wherein
M is a metal ion selected from a main group metal or transition metal, preferably Cu, Fe, Mn, Al, Hf, Zr;
n is the valency of M and selected from 2 or 3;
L has formula (II) 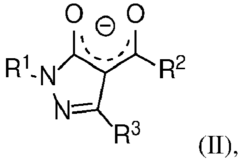
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
R³ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprises at least three atoms selected from the group consisting of halogen, Cl, F or N;
wherein
the group consisting of R¹, R² and R³ comprises in addition:
- at least two moieties selected from CF₃ or CN; or
- at least one moiety selected from CF₃ or CN and one substituted or unsubstituted 6-membered heteroaryl; or
- R³ is CN;
AL is an ancillary ligand which coordinates to the metal M;
m is an integer selected from 0 to 2.

14. The metal complex of formula (I) according to claims 1 to 13:
M^{n_{⊕}}(L^{Θ})n(AL)m (I),
wherein
M is a Ce ion;
n is the valency of M and is 4;
L has formula (II) 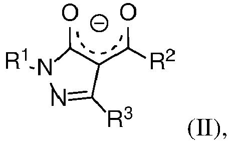
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
R³ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprise at least three atoms selected from the group consisting of halogen, Cl, F or N;
AL is an ancillary ligand which coordinates to the metal M;
m is an integer selected from 0 to 2.

15. The metal complex of formula (I) according to claims 1 to 14:
M^{n_{⊕}}(L^{Θ})n(AL)m (I),
wherein
M is a metal ion;
n is the valency of M and selected from 1 to 4;
L has formula (II) 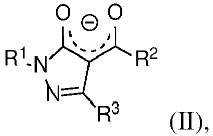
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl that comprises at least one substituted or unsubstituted 6-membered heteroaryl;
R³ is selected from partially or perfluorinated C₁ to C₄ alkyl or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
AL is an ancillary ligand which coordinates to the metal M;
m is an integer selected from 0 to 2.

16. The metal complex of formula (I) according to claims 1 to 15:
M^{n_{⊕}}(L^{Θ})n(AL)m (I),
wherein
M is a metal ion;
n is the valency of M and selected from 1 to 4;
L has formula (II) 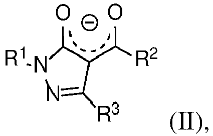
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl;
R³ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprises a substituted or unsubstituted C₂ to C₂₀ heteroaryl that comprises at least one 6 membered heteroaryl ring;
wherein
AL is an ancillary ligand which coordinates to the metal M;
m is an integer selected from 0 to 2.

17. A semiconductor material comprising at least one metal complex of Formula (I) according to any of the preceding claims 1 to 16.

18. A semiconductor material according to claim 17, wherein the semiconductor material comprises in addition at least one covalent matrix compound, preferably at least one substantially covalent matrix compound.

19. An organic electronic device comprising a first electrode layer, a second electrode layer, at least one photoactive layer and a semiconductor layer, wherein the semiconductor layer is arranged between the first electrode layer and the at least one photoactive layer, the semiconductor layer comprises a metal complex of formula (I)
M^{n_{⊕}}(L^{Θ})n(AL)m (I),
wherein
M is a metal ion;
n is the valency of M and selected from 1 to 4;
L has formula (II) 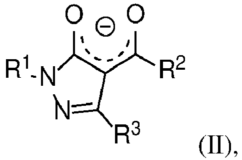
R¹ and R² are independently selected from substituted or unsubstituted C₁ to C₁₂ alkyl, substituted or unsubstituted C₆ to C₁₉ aryl, substituted or unsubstituted C₂ to C₂₀ heteroaryl, or substituted or unsubstituted 6-membered heteroaryl;
R³ is selected from substituted or unsubstituted C₁ to C₁₂ alkyl, substituted or unsubstituted C₆ to C₁₉ aryl, substituted or unsubstituted C₂ to C₂₀ heteroaryl, substituted or unsubstituted 6-membered heteroaryl, or CN;
wherein
at least one of the substituents of the substituted C₁ to C₁₂ alkyl, substituted C₆ to C₁₉ aryl, substituted C₂ to C₂₀ heteroaryl, or substituted 6-membered heteroaryl are independently selected from halogen, Cl, F, CN, partially or perfluorinated C₁ to C₈ alkyl, partially or perfluorinated C₁ to C₈ alkoxy;
wherein
at least one of R¹, R² and R³ or the group of R¹, R² and R³ comprises at least three atoms selected from the group consisting of halogen, Cl, F or N;
AL is an ancillary ligand which coordinates to the metal M;
m is an integer selected from 0 to 2.

20. The organic electronic device according to claim 19, wherein the semiconductor layer comprises a metal complex of formula (I) according to claims 1 to 16.

21. The organic electronic device according to claim 19 or 20, wherein the organic electronic device is preferably a light emitting device, a thin film transistor, a battery, a display device or a photovoltaic device, and preferably a light emitting device, preferably the electronic device is part of a display device or lighting device.

22. An organic electronic device comprising a metal complex of formula (I) according to claims 1 to 16, wherein the organic electronic device is preferably a light emitting device, thin film transistor, a battery, a display device or a photovoltaic device, and preferably a light emitting device, preferably the electronic device is part of a display device or lighting device.
